# EUROPEAN PATENT SPECIFICATION

(11) **EP 3 087 181 B1**
(45) Date of publication and mention of the grant of the patent: **02.12.2020**
(21) Application number: 14833321.4
(22) Date of filing: 23.12.2014
(51) Int. Cl.: C12N 15/09

(54) **STRUCTURED SUBSTRATES FOR IMPROVING DETECTION OF LIGHT EMISSIONS AND METHODS RELATING TO THE SAME**
STRUKTURIERTE SUBSTRATE ZUR VERBESSERTEN DETEKTION VON LICHTEMISSIONEN UND VERFAHREN IM ZUSAMMENHANG DAMIT
SUBSTRATS STRUCTURÉS PERMETTANT D'AMÉLIORER LA DÉTECTION DES ÉMISSIONS DE LUMIÈRE ET PROCÉDÉS SE RAPPORTANT À CES DERNIERS

(30) Priority: 23.12.2013 US 201361920244 P
(43) Date of publication of application: 02.11.2016
(60) Divisional application: 20200233.3
(73) Proprietor: Illumina, Inc., San Diego, CA 92122 (US)
(72) Inventor: BOWEN, M., Shane, San Diego, CA 92122 (US); VENKATESAN, Bala, Murali, San Diego, CA 92122 (US); HAN, Hui, San Diego, CA 92122 (US); PARK, Sang, Ryul, San Diego, CA 92122 (US)
(74) Representative: Murgitroyd & Company
(86) International application number: PCT/US2014/072256
(87) International publication number: WO 2015/100373

(56) References cited:
- WO-A2-03/049677
- WO-A2-2013/063382
- US-A1- 2003 059 929
- VENKATESAN BALA MURALI ET AL: "Nanopore sensors for nucleic acid analysis.", NATURE NANOTECHNOLOGY OCT 2011, vol. 6, no. 10, October 2011 (2011-10), pages 615-624, XP002739803, ISSN: 1748-3395

## Description

### BACKGROUND

The present disclosure relates generally to biological or chemical analysis and more particularly to systems and methods for detecting light emissions from reaction sites.

Various protocols in biological or chemical research involve performing a large number of controlled reactions at localized areas of a support surface or within reaction cavities. The designated reactions may then be observed or detected and subsequent analysis may help identify or reveal properties of chemicals involved in the reaction. For example, in some multiplex assays, an unknown analyte having an identifiable label (e.g., fluorescent label) may be exposed to thousands of known probes under controlled conditions. Each known probe may be deposited into a corresponding well of a microplate. Observing any chemical reactions that occur between the known probes and the unknown analyte within the wells may help identify or reveal properties of the analyte.

Other protocols that detect light emissions from an array of reaction sites include known DNA sequencing protocols, such as sequencing-by-synthesis (SBS) or cyclic-array sequencing. In SBS, a plurality of fluorescently-labeled nucleotides are used to sequence nucleic acids of numerous clusters (or clonal populations) of amplified DNA that are located on the surface of a substrate. The surface may, for example, define a channel in a flow cell. The sequences of the nucleic acids in the different clusters are determined by running numerous cycles in which a fluorescently-labeled nucleotide is added to the cluster and then excited by a light source to provide light emissions.

Although the sequencing systems currently used are effective in identifying the nucleotides and determining a sequence of the nucleic acids, systems that are more cost-effective and/or that achieve an even smaller error rate are desired. For example, it is desirable to increase the density of reaction sites. Sequencing methodologies and corresponding systems, however, exploit a complex collection of technologies. Improvements in some of these technologies have been shown to provide substantial cost reductions. However, it is difficult to predict which, if any, is amenable to cost-reducing improvements. Given the dependencies between the technologies in the sequencing systems it is even more difficult to predict which can be modified without having an adverse impact on the overall performance of the methodology or system.

One challenge confronted by many systems and protocols is detecting, with a suitable level of confidence, the designated reactions that generate light emissions. This challenge is even more difficult as the reaction sites become smaller and the density of reaction sites becomes greater. One consequence of the reaction sites becoming smaller is that the amount of generated light emissions also becomes smaller. Moreover, as the density of reaction sites becomes greater, it may be more difficult to distinguish which reaction sites provided the light emissions. In addition to the above, it is generally desirable to decrease the amount of time used for detecting the light emissions (also referred to as scan time or image time). As scan times decrease, fewer photons are detected, thereby rendering it even more challenging to reliably detect light emissions that are indicative of a designated reaction occurring.

Accordingly, a need exists for apparatuses, systems, and methods that generate a sufficient amount of light for detecting designated reactions within an array of reaction sites.

### BRIEF SUMMARY

Presented herein are structures substrates and methods for manufacturing structures substrates that improve the detectability of optical emissions provided by discrete reaction sites. For example, the structures substrates may increase an intensity of an excitation light experienced by biological substances at the discrete sites, may increase an intensity of the optical emissions from the biological substances, and/or may control a directionality of the optical emissions. Also presented herein are methods of detecting optical emissions from an array of discrete sites. The discrete sites may be reaction cavities formed within a substrate body or localized areas along a surface of a device substrate. The optical emissions may be generated by, for example, fluorescence, chemiluminescence, bioluminescence, electroluminescence, radioluminescence, and the like. Also presented herein are structured substrates having a greater density of discrete sites (or smaller pitch between adjacent sites) than known systems and methods of manufacturing the same.

The methods and structured substrates may be configured to enhance the light emissions of fluorescently-labeled samples and, more specifically, fluorescently-labeled nucleic acids. The methods and compositions presented herein provide fluorescent enhancement of DNA clusters in sequencing by synthesis reactions involving dye-labeled nucleotides.

Methods and compositions for fluorescent enhancement on a surface are provided. The methods and compositions are well suited for enhancing the fluorescence intensity of labeled nucleic acids on a solid support. The methods and compositions presented herein provide fluorescent enhancement of DNA clusters in sequencing by synthesis reactions involving dye-labeled nucleotides.

Accordingly, presented herein is a substrate, comprising: a plurality of nanoparticles distributed on a solid support; a gel material forming a layer in association with the plurality of nanoparticles; and a library of target nucleic acids in the gel material. The nanoparticles can be formed of a plasmon resonant material. The plasmon resonant material can comprise a material selected from the group consisting of: Gold (Au), Silver (Ag), Tin (Sn) Rhodium (Rh), Ruthenium (Ru), Palladium (Pd), Osmium (Os), Iridium (Ir), Platinum (Pt), Titanium (Ti) and Aluminum (Al), Chromium (Cr), Copper (Cu), Silicon (Si) (e.g., p-type doped silicon, n-type doped silicon), and gallium arsenide. Gel material can cover the nanoparticles. The solid support can comprise a surface of a flow cell. The solid support can comprise a planar surface having a plurality of wells (or reaction cavities), the nanoparticles being distributed within the plurality of wells.

Also presented herein is a method of making a substrate, comprising: (a) providing a solid support comprising a planar surface; (b) dispersing a plurality of nanoparticles on the surface of the solid support; (c) and coating at least a portion of the solid support with a gel material thereby forming a gel layer covering the plurality of nanoparticles. The nanoparticles can be formed of a plasmon resonant material. Steps (b) and (c) can be performed simultaneously. Step (b) can be performed prior to step (c). The method can further comprise (d) delivering a library of target nucleic acids to the gel material to produce an array of nucleic acid features in the gel material. Each feature can comprise a different nucleic acid species. The plasmon resonant material can comprise a material selected from the group consisting of: Gold (Au), Silver (Ag), Tin (Sn) Rhodium (Rh), Ruthenium (Ru), Palladium (Pd), Osmium (Os), Iridium (Ir), Platinum (Pt), Titanium (Ti) and Aluminum (Al), Chromium (Cr), Copper (Cu), Silicon (SI) (e.g., p-type doped silicon, n-type doped silicon), and gallium arsenide.

Also presented herein is a method of detecting nucleic acids, comprising: providing a solid support comprising a plurality of nanoparticles; a gel material forming a layer covering the plurality of nanoparticles; and a library of target nucleic acids in the gel material; contacting the solid support with at least one fluorescently labeled probe that binds to the target nucleic acids; and detecting fluorescent signal on the solid support to distinguish the target nucleic acids that bind to the at least one probe. The nanoparticles can be formed of a plasmon resonant material. The plasmon resonant material can comprise a material selected from the group consisting of: Gold (Au), Silver (Ag), Tin (Sn) Rhodium (Rh), Ruthenium (Ru), Palladium (Pd), Osmium (Os), Iridium (Ir), Platinum (Pt), Titanium (Ti) and Aluminum (Al), Chromium (Cr), Copper (Cu), Silicon (Si) (e.g., p-type doped silicon, n-type doped silicon), and gallium arsenide. The solid support can comprise a surface of a flow cell. The solid support can comprise planar surface having a plurality of wells (or reaction cavities), the nanoparticles distributed among the plurality of wells. The fluorescently labeled probe can comprise a fluorescently labeled nucleotide. The fluorescently labeled probe can comprise a fluorescently labeled oligonucleotide. Detecting can comprise detection of hybridization of an oligonucleotide probe to target nucleic acids in each feature. Detecting can comprise detection of incorporation of a nucleotide or an oligonucleotide probe to target nucleic acids in each feature.

The invention presented herein is an array, comprising: a solid support comprising a surface, the surface comprising a plurality of wells (or reaction cavities), the wells being separated from each other by interstitial regions; a plurality of plasmonic nanostructures in each of said plurality of wells; a gel material forming a layer on the plurality of plasmonic nanostructures; and a primer nucleic acid attached to the gel material In certain embodiments, the nanostructures are situated at the bottom of the wells. In certain embodiments, the nanostructures are situated along the walls of the wells. In certain embodiments, the interstitial regions are substantially devoid of nanostructures. In certain embodiments, the nanostructures comprise nanoparticles. In certain embodiments, the nanoparticles have a diameter of greater than 1 nm, 2 nm, 3 nm, 4 nm, 5 nm, 6 nm, 7 nm, 8 nm, 9 nm, 10 nm, 20 nm, 30 nm, 40 nm, 50 nm, 60 nm, 70 nm, 80 nm, 90 nm or greater than 100 nm. In certain embodiments, the nanoparticles have a diameter of less than 100 nm, 90 nm, 80 nm, 70 nm, 60 nm, 50 nm, 40 nm, 30 nm, 20 nm, 10 nm, 9 nm, 8 nm, 7 nm, 6 nm, 5 nm, 4 nm, 3 nm, 2 nm, or less than 1 nm. In certain embodiments, the nanoparticles comprise dimers or trimers within the wells. In certain embodiments, the nanostructures comprise bowtie nanoantennae. In certain embodiments, the nanostructures comprise nanorods. In certain embodiments, the nanostructures comprise nanorings. In certain embodiments, the nanostructures comprise nanoplugs. In certain embodiments, the nanostructures comprise nanogratings. In certain embodiments, the gel material comprises a hydrogel. In certain embodiments, the solid support comprises a surface of a flow cell.

Also presented herein is a method of making an array, comprising obtaining a solid support comprising a planar surface, the surface comprising a plurality of wells (or reaction cavities), the wells being separated from each other by interstitial regions; coating a metal film on the solid support; subjecting the metal film to a thermal annealing process, thereby forming a plurality of nanostructures in each of said plurality of wells; coating at least a portion of the solid support with a gel material, thereby depositing the gel material in a plurality of the wells and comprising a primer nucleic acid attached to the gel. In certain embodiments, the nanostructures are formed of a plasmon resonant material. In certain embodiments, the method further comprises polishing the planar surface to substantially remove nanostructures from the interstitial regions and to maintain the nanostructures in the wells. In certain embodiments, the method further comprises coating at least a portion of the solid support with a gel material, thereby depositing the gel material in a plurality of the wells. In certain embodiments, nanostructures comprise a material selected from the group consisting of: Gold (Au), Silver (Ag), Tin (Sn) Rhodium (Rh), Ruthenium (Ru), Palladium (Pd), Osmium (Os), Iridium (Ir), Platinum (Pt), Titanium (Ti) and Aluminum (Al), Chromium (Cr), Copper (Cu), Silicon (Si) (e.g., p-type doped silicon, n-type doped silicon), and gallium arsenide.

Also presented herein is a method of detecting nucleic acids, comprising: providing a solid support comprising a planar surface, the surface comprising a plurality of wells, the wells being separated from each other by interstitial regions; a plurality of plasmonic nanostructures in each of said plurality of wells; a gel material forming a layer covering the plurality of plasmonic nanostructures; a primer nucleic acid attached to the gel material; and a library of target nucleic acids that seed individual wells via interaction with the primer nucleic acid attached to the gel material; contacting the solid support with at least one fluorescently labeled probe that binds to the target nucleic acids; and detecting fluorescent signal on the solid support to distinguish the target nucleic acids that bind to the at least one probe. In certain embodiments, the nanostructures are formed of a plasmon resonant material. In certain embodiments, nanostructures comprise a material selected from the group consisting of: Gold (Au), Silver (Ag), Tin (Sn) Rhodium (Rh), Ruthenium (Ru), Palladium (Pd), Osmium (Os), Iridium (Ir), Platinum (Pt), Titanium (Ti) and Aluminum (Al), Chromium (Cr), Copper (Cu), Silicon (Si) (e.g., p-type doped silicon, n-type doped silicon), and gallium arsenide. In certain embodiments, the nanostructures are situated at the bottom of the wells. In certain embodiments, the nanostructures are situated along the walls of the wells. In certain embodiments, the interstitial regions are substantially devoid of nanostructures. In certain embodiments, the gel material comprises a hydrogel. In certain embodiments, the solid support comprises a surface of a flow cell. In certain embodiments, the fluorescently labeled probe comprises a fluorescently labeled nucleotide. In certain embodiments, the fluorescently labeled probe comprises a fluorescently labeled oligonucleotide. In certain embodiments, detecting comprises detection of hybridization of an oligonucleotide probe to target nucleic acids in each feature. In certain embodiments, detecting comprises detection of incorporation of a nucleotide or an oligonucleotide probe to target nucleic acids in each feature.

A structured substrate is provided. The structured substrate includes a substrate body having an active side. The substrate body includes reaction cavities that open along the active side and interstitial regions that separate the reaction cavities. The structured substrate includes an ensemble amplifier positioned within each of the reaction cavities. The ensemble amplifier includes a plurality of nanostructures configured to at least one of amplify electromagnetic energy that propagates into the corresponding reaction cavity or amplify electromagnetic energy that is generated within the corresponding reaction cavity.

A method of manufacturing a structured substrate is provided The method includes providing a base layer having a base side and forming nanostructures along the base side of the base layer. The method also includes forming a cavity layer that is stacked above the base side. The cavity layer includes a plurality of reaction cavities in which each reaction cavity includes a plurality of the nanostructures therein. The plurality of nanostructures form an ensemble amplifier of the corresponding reaction cavity that is configured to at least one of amplify electromagnetic energy propagating into the corresponding reaction cavity or amplify electromagnetic energy generated within the corresponding reaction cavity

A method of manufacturing a structured substrate is provided. The method includes providing a base layer having a base side and forming nanostructures along the base side of the base layer. The method also includes providing a nanoimprint lithography (NIL) layer over the array of nanostructures. The method also includes imprinting an array of reaction cavities into the NIL layer, wherein a different sub-array of the nanostructures is positioned under each reaction cavity. Each sub-array of nanostructures being surrounded by a respective fill region of the NIL layer. The method also includes removing the respective fill regions of the NIL layer to expose the sub-arrays of nanostructures within the corresponding reactions cavities. The sub-array of nanostructures within each reaction cavity forming an ensemble amplifier of the corresponding reaction cavity that is configured to at least one of amplify electromagnetic energy propagating into the corresponding reaction cavity or amplify electromagnetic energy generated within the corresponding reaction cavity.

A method of manufacturing a structured substrate is provided. The method includes providing a base layer having a base side and providing a nanoimprint lithography (NIL) layer along the base side. The method also includes imprinting the NIL layer to form a base portion and an array of nano-bodies that project from the base portion. The method also includes depositing a plasmon resonant film that covers the nano-bodies to form a plurality of nanostructures. Each nanostructure including a corresponding nano-body and a portion of the plasmon resonant film . The method also includes forming a cavity layer including a plurality of reaction cavities in which each reaction cavity includes a plurality of the nanostructures therein. The plurality of nanostructures form an ensemble amplifier of the corresponding reaction cavity that is configured to at least one of amplify electromagnetic energy propagating into the corresponding reaction cavity or amplify electromagnetic energy generated within the corresponding reaction cavity.

The details of one or more embodiments are set forth in the accompanying drawings and the description below. Other advantages will be apparent from the description and drawings, and from the claims.

### BRIEF DESCRIPTION OF THE DRAWINGS

Figure 1 is a schematic showing an exemplary method for fluorescent enhancement on a surface.
Figure 2 is a diagrammatical representation of several examples of plasmonic nanostructures in nanowells.
Figure 3A shows a schematic of deposition of nanoparticles in nanowells. Figures 3B and 3C are SEM images showing deposition of nanoparticles at various thicknesses.
Figure 4 shows a schematic and SEM images showing formation of nanorings in nanowells.
Figure 5 is an SEM image of nanoparticles deposited in nanowells following thermal annealing. Scale bar is 2 µm.
Figure 6 is a set of graphs showing cluster intensity per cycle for four fluorescently labeled bases as a function of Sn/Au particle thickness.
Figure 7 is a summary table of cluster intensity enhancement at cycle 1 and cycle 26 for annealed Sn/Au nanoparticle films.
Figure 8A is a schematic showing formation of Au nanoplugs in nanowells according to one embodiment. Figure 8B is a SEM image demonstrating formation of Au nanoplugs.
Figure 9A is a bar graph showing enhancement of first cycle sequencing intensity using Au nanoplugs in nanowells according to one embodiment. Figure 9B is a summary table of cluster intensity enhancement for annealed Au nanoplugs in nanowells.
Figure 10 illustrates a cross-section of a portion of a structured substrate formed in accordance with an embodiment.
Figure 11 is a flow chart illustrating a method of manufacturing a structured substrate in accordance with an embodiment.
Figure 12 is a flow chart illustrating a method of manufacturing a structured substrate in accordance with an embodiment that includes nano-imprint lithography (NIL) material.
Figure 13 illustrates different steps of the method shown in Figure 12.
Figure 14 illustrates different steps of the method shown in Figure 12.
Figure 15 is a flow chart illustrating a method of manufacturing a structured substrate in accordance with an embodiment that includes NIL material.
Figure 16 illustrates different steps of the method shown in Figure 15.
Figures 17A-17E illustrate perspective views of nanostructures that may be used with one or more embodiments.
Figures 18A-18D illustrate cross-sections of nanostructures that may be used with one or more embodiments.
Figures 19A-19D illustrate plan views of nanostructures that may be used with one or more embodiments.

### DETAILED DESCRIPTION

The application cites the following prior art U.S. Patent Appl. Publ. No. 2014/0242334; U.S. Patent Appl. Publ. No. 2014/0079923; and U.S. Patent Appl. Publ. No. 2011/0059865.

One or more embodiments set forth herein are configured to directly or indirectly enhance light emissions from an array of reaction sites so that the light emissions may be detected by, for example, an imaging system or device. To this end, embodiments may at least one of increase an intensity of an excitation light experienced by a biological substance, increase an intensity of the light emissions generated by the biological substance, and/or control a directionality of the light emissions so that the light emissions may be detected. The increase in intensity and/or control of the directionality of the light emissions may be caused, in part, by one or more nanostructures located at the corresponding reaction site. The amount of increase may be measured relative to an amount of electromagnetic energy that exists at the reaction site without the nanostructure(s).

The array of reaction sites may be disposed along a structured substrate. The structured substrate may be, for example, a flow cell having a channel for directing reagents alongside the reaction sites. The light emissions may be detected by an imaging system that may include, for example, an objective lens that scans or sweeps alongside the structured substrate to detect the light emissions from the reaction sites. Exemplary systems capable of detecting light emissions from the structured substrates set forth herein are described in U.S. Appl. Publ. Nos. 2012/0270305 A1 and 2013/0261028 A1. Alternatively, the structured substrate may be integrated with an imaging device, such as a solid-state imaging device (e.g., CMOS). In such embodiments, the imaging device may have one or more light sensors that are aligned with reaction sites to capture light emissions from the reaction sites. Such embodiments are described in U.S. Provisional Application No. 61/914,275 and International Application No. PCT/US14/69373.

A technical effect provided by at least one of the embodiments may include an increased signal intensity from the emitters of the biological substance. The increase in signal intensity may reduce an error rate by reducing the number of biological substances that emit a low intensity of light. Another technical effect may include a decrease in signal to noise ratio that enables faster scan speeds and reduces overall time for conducting a protocol. For instance, with respect to sequencing-by-synthesis technology, faster scan speeds on sequencing instruments are desired, but faster scan speeds result in fewer photons being collected per cluster on the imaging camera. With fewer photons captured, the signal to noise ratio typically decreases and it becomes more difficult to confidently assign a base. Furthermore on some sequencing instruments, low NA optics result in signals that arc inherently larger and dimmer, potentially yielding higher error rates. Embodiments set forth herein may increase the number of photons that are captured. Another technical effect for at least some embodiments includes a method of manufacturing a structured substrate that is more reliable than at least some known methods and more cost-effective than at least some known methods.

The present disclosure relates generally to solid-phase analytical chemistry, and has specific applicability to nucleic acid arrays for high throughput genomics analysis. The task of cataloguing human genetic variation and correlating this variation with susceptibility to disease stands to benefit from advances in genome wide sequencing methodologies. This cataloguing effort holds promise for identifying the markers in each person's genome that will help medical professionals determine susceptibility of that person to disease, responsiveness to specific therapies such as prescription drugs, susceptibility to dangerous drug side effects and other medically actionable characteristics. The cataloguing effort is well under way. This is due in large part to commercially available genome sequencing methodologies which are sufficiently cost effective to allow test subjects to be evaluated in a research setting. Improvements in sequencing methodologies are needed to accelerate the cataloguing effort. Moreover, the relatively high cost of sequencing has hindered the technology from moving beyond the research centers and into the clinic where doctors can obtain sequences for patients in the general population.

Sequencing methodologies and the systems used to carry them out, exploit a complex collection of technologies. Improvements in some of these technologies have been shown to provide substantial cost reductions. However, it is difficult to predict which if any is amenable to cost reducing improvements. Given the dependencies between the technologies in the sequencing systems it is even more difficult to predict which can be modified without having an adverse impact on the overall performance of the methodology or system. Thus, there exists a need to identify improvements that can bring the promise of genomics research to the clinic where lives can be improved and in many cases saved. The present invention satisfies this need and provides related advantages as well.

As used herein, a "biological substance" or "chemical substance" includes biomolecules, samples-of-interest, analytes-of-interest, and other chemical compound(s). A biological or chemical substance may be used to detect, identify, or analyze other chemical compound(s), or function as intermediaries to study or analyze other chemical compound(s). In particular embodiments, the biological substance is a nucleic acid or, more specifically, a colony of nucleic acids having a common sequence. In particular embodiments, the biological or chemical substances include a biomolecule. As used herein, a "biomolecule" includes at least one of a biopolymer, nucleoside, nucleic acid, polynucleotide, oligonucleotide, protein, enzyme, polypeptide, antibody, antigen, ligand, receptor, polysaccharide, carbohydrate, polyphosphate, cell, tissue, organism, or fragment thereof or any other biologically active chemical compound(s) such as analogs or mimetics of the aforementioned species.

As another example, a biological or chemical substance may include an enzyme or reagent used in a coupled reaction to detect the product of another reaction such as an enzyme or reagent used to detect pyrophosphate in a pyrosequencing reaction. Enzymes and reagents useful for pyrophosphate detection are described, for example, in U.S. Patent Publication No. 2005/0244870 A1.

Biological or chemical substances may be naturally occurring or synthetic and located within a designated area or space. In some embodiments, the biological or chemical substances may be bound to a solid phase or gel material. Biomolecules, samples, and biological or chemical substances may also include a pharmaceutical composition. In some cases, biomolecules, samples, and biological or chemical substances of interest may be referred to as targets, probes, or analytes.

Embodiments may be particularly suitable for enhancing emissions from fluorescently-labeled nucleic acids. By way of example, embodiments may provide fluorescent enhancement of DNA clusters in sequencing by synthesis reactions involving dye-labeled nucleotides. Embodiments may increase a signal intensity from flurorescent labels during sequencing by synthesis. The increase in signal intensity may improve overall sequencing performance by reducing sequencing error arising from low intensity clusters and cluster dropouts during long sequencing runs.

Presented herein are methods and compositions for fluorescent enhancement on a surface. The methods and compositions are well suited for enhancing the fluorescence intensity of labeled nucleic acids on a solid support (or substrate body). In particular embodiments, the methods and compositions presented herein provide fluorescent enhancement of DNA clusters in sequencing by synthesis reactions involving dye-labeled nucleotides. Additionally, provided herein are methods for low-cost, rapid and robust fabrication of nanostructures such as plasmonic nanostructures on sequencing substrates capable of broad spectrum fluorescence enhancement from about 350nm to about 750nm.

The present disclosure details the surprising discovery that increased intensity from clustered nucleic acids can be obtained by combining plasmonics and/or nanoantennae with exemplary sequencing substrates/platforms and SBS chemistry. This is achieved either through the top-down nanofabrication or bottom up self-assembly of plasmonic nanostructures and nano-antennae on the sequencing substrate. A variety of methods for fabricating plasmonic nanostructures on both non-patterned and patterned substrates are presented herein.

Without wishing to be bound by theory, the resulting enhancement in cluster intensity is due to a combination of localized surface plasmon resonance and resonant energy transfer processes. The methods and compositions presented herein have several advantages. For example, increased signal intensity from flurorescent labels during sequencing by synthesis improves overall sequencing performance by reducing sequencing error arising from low intensity clusters and cluster dropouts during long sequencing runs. Furthermore, signal to noise ratio is decreased thereby enabling faster scan speeds and reducing overall sequencing run time. Faster scan speeds on sequencing instruments are desired, but faster scan speeds result in fewer photons being collected per cluster on the imaging camera resulting in lower signal to noise and higher error rates. Furthermore on some sequencing instruments, low NA optics result in signals that are inherently larger and dimmer, potentially yielding higher error rates. Therefore, methods to increase cluster intensity provide numerous benefits.

Presented herein is a substrate, comprising: a plurality of nanoparticles formed of a plasmon resonant material distributed on a solid support (or substrate body); a gel material forming a layer in association with the plurality of nanoparticles; and a library of target nucleic acids in the gel material.

Gel material can cover the nanoparticles. The solid support can comprise a surface of a flow cell. The solid support can comprise a planar surface having a plurality of wells (or reaction cavities), the nanoparticles being distributed within the plurality of wells.

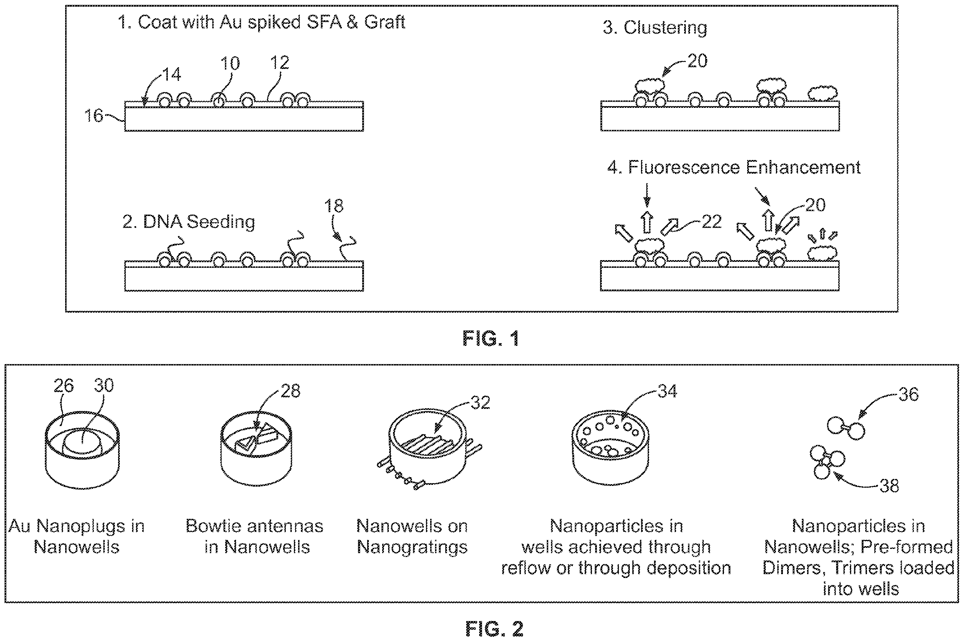

As used herein, the terms "nanoparticle" and "nanostructure" are used interchangeably to refer to a particle having one dimension in the range of about 1 nm to about 1000 nm, including any integer or non-integer value between 1 nm and 1000 nm. In typical embodiments, the nanoparticle is a metallic particle. In some embodiments, the nanoparticle core is a spherical or nearly spherical particle of 20-200 nm in diameter. In some embodiments the range is about 1 nm to about 50 nm (for example about 1, 2, 3, 4, 5, 6, 7, 8, 9, 10 , 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 nm). In some embodiments the range is about 350 nm to about 750 nm. Anisotropic nanoparticles may have a length and a width. In some embodiments, the length of an anisotropic nanoparticle is a dimension parallel to the plane of the aperture in which the nanoparticle was produced. In some embodiments, the length of an anisotropic nanoparticle is the dimension perpendicular to the plane of the aperture in which the nanoparticle was produced. In the case of anisotropic nanoparticles, in some embodiments, the nanoparticle has a diameter (width) in the range of about 350 nm to about 750 nm. In other embodiments, the nanoparticle has a diameter (width) of about 350 nm or less. In other embodiments, the nanoparticle has a diameter of 250 nm or less and in some embodiments, a diameter of 100 nm or less. In some embodiments, the width is between 15 nm to 300 nm. In some embodiments, the nanoparticle has a length of about 10-350 nm. In some embodiments, the nanoparticles have a preselected shape and can be, for example a nanotube, a nanowire, nanosphere, or any shape comprising the above-described dimensions (e.g., triangular, square, rectangular, or polygonal shape in 2 dimensions, or cuboid, pyramidal, cylindrical, spherical, discoid, or hemispheric shapes in the 3 dimensions). Figure 2 is a diagrammatical representation of several examples of plasmonic nanostructures in nanowells 26. Some examples of nanostructures include, for example, bowtie nanoantennae 28, nanospheres 34, nanopyramids, nanoshells, nanorods, nanowires, nanorings, nanoplugs 30, nanogratings 32 and the like. Preformed dimers 36 and trimers 28 of nanoparticles can also be loaded into wells and have the advantage of precisely controlling nanoparticle spacing.

These nanostructures can either be fabricated on a surface or pre-formed and then loaded into nanowells. Examples of such structures include plasmonic nanoplugs fabricated at the bottom of nanowells, bowtie and cavity antennas in nanowells, metal nanogratings on which nanowells could be formed, nanoparticles reflowed in nanowells or a combination of some or all of the above. One example would be a metal nanoplug in a nanowell with nanoparticles on the walls formed through a simple electron beam evaporation process. Nanoparticle constructs (dimers, n-mers) formed using DNA origamis or linker molecules such as cucurbit[n]urils are also attractive for loading into wells. Such methods allow for precise subnanometer control over nanoparticle spacings and can be formed on a large scale using bottom up self-assembly. Examples of these structures are shown in Figure 2.

The spacing between any two nanoparticles on a surface can be any distance. In some embodiments, the spacing can be a multiple of a wavelength of incident light energy, such as a particular emission or excitation wavelength in fluorescence spectroscopy. The spacing can be, for example, 1 λ, 2 λ, 3 λ, 4 λ or another multiple of a chosen wavelength (λ) of incident light energy. Thus, using as an example an emission wavelength (λ) of 532 nm, the spacing between nanoparticles can be about 532 nm (1 λ), about 1064 nm (2 λ), or another multiple of the emission wavelength. In some embodiments, the spacing can be a fraction of a wavelength of incident light energy, such as a particular emission or excitation wavelength in fluorescence spectroscopy. The spacing can be, for example, 1 λ, ½ λ, 1/3 λ, 1/4 λ or another multiple of a chosen wavelength of incident light energy. Thus, using as an example an emission wavelength of (λ) 532 nm, the spacing between nanoparticles can be about 532 nm (1 λ), 266 nm (1/2 λ), 133 nm (1/3 λ) or another fraction of the emission wavelength.

The terms "plasmonic nanostructure" or "nanoplasmonic structure" are used interchangeably herein and refer to any independent structure exhibiting plasmon resonance characteristic of the structure, including (but not limited to) both nanostructures, nanoparticles and combinations or associations of nanoparticles.

The term "nanoantenna" as used herein refers to a nanoparticle or nanostructure that acts to amplify electromagnetic energy, such as light energy. As used herein, a nanoantenna does not necessarily exhibit plasmon resonance characteristics. In some embodiments, a nanoantenna does not substantially comprise a plasmon resonant material. Thus, in some embodiments, nanoantennas are presented which are made of a non-metal material but which exhibits amplification characteristics of electromagnetic energy. Nanoparticles presented herein can be of any suitable shape and size so as to produce the desired energy amplification. Some exemplary shapes of nanoantennas include, for example, bowtie nanoantennae, nanospheres, nanopyramids, nanoshells, nanorods, nanowires, nanorings, nanoplugs, nanogratings and the like. It will be appreciated that any of a number of known methods can be suitable for fabrication and/or deposition of nanoantenna on a solid support (or substrate body). Methods for fabrication of nanoantenna are known in the art and include, for example, the methods described herein for nanoparticle fabrication and deposition.

The nanoparticles can comprise any material suitable for use in the methods and compositions described herein, for example, any type of material exhibiting surface plasmon resonance (SPR). In certain preferred embodiments, the nanoparticle comprises a plasmon resonant material. Examples include, but are not limited to, metal nanoparticles. For example, the nanoparticles can comprise a metal such as one or more of Gold (Au), Silver (Ag), Tin (Sn) Rhodium (Rh), Ruthenium (Ru), Palladium (Pd), Osmium (Os), Iridium (Ir), Platinum (Pt), Titanium (Ti) and Aluminum (Al), Chromium (Cr), Copper (Cu), or any other suitable metal. The nanoparticles can be formed from a single material such as, for example a single metal. Additionally or alternatively, the nanoparticles can be formed from a combination of two or more different materials, such as, for example, two or more metals. For example, the nanoparticles can comprise a metal/metal mixture such as Sn/Au or Ag/Au. Alternatively or additionally, vertical layered nanoparticles, such as multilayer structures of the metal-insulator-metal type may be applied. Examples include Silicon (Si). For instance, the nanoparticles may include p-type doped silicon and n-type doped silicon. Gallium arsenide may also be used.

Formation of nanoparticles on a solid support can be performed using any one of a number of methods known in the art. Nanoparticles can be formed using bottom-up self-assembly of plasmonic nanostructures and nano-antennae on the sequencing substrate. For example, any one of a number of methods for deposition of layers of a material can be used, such as those described by Gaspar et al. (Scientific Reports, 2013, 3, 1469). In exemplary embodiments described herein and illustrated in Example 1 and Figure 1, nanoparticles 10 can be pre-formed and mixed in a colloid-like

composition with a gel material 12, which is deposited on a surface 14 of a solid support 16. A seeding operation may occur in which nucleic acids 18 (or other biomoleclues) are immobilized to the gel material 12. A clustering operation may be performed to form a cluster 20 of the nucleic acids 18. The clusters may be generated, for example, through bridge amplification. Alternatively or additionally, nanoparticles can be first deposited on a surface followed by deposition of a gel material over the nanoparticles. In other embodiments, a gel material can be deposited on a surface and nanoparticles are deposited over the gel material.

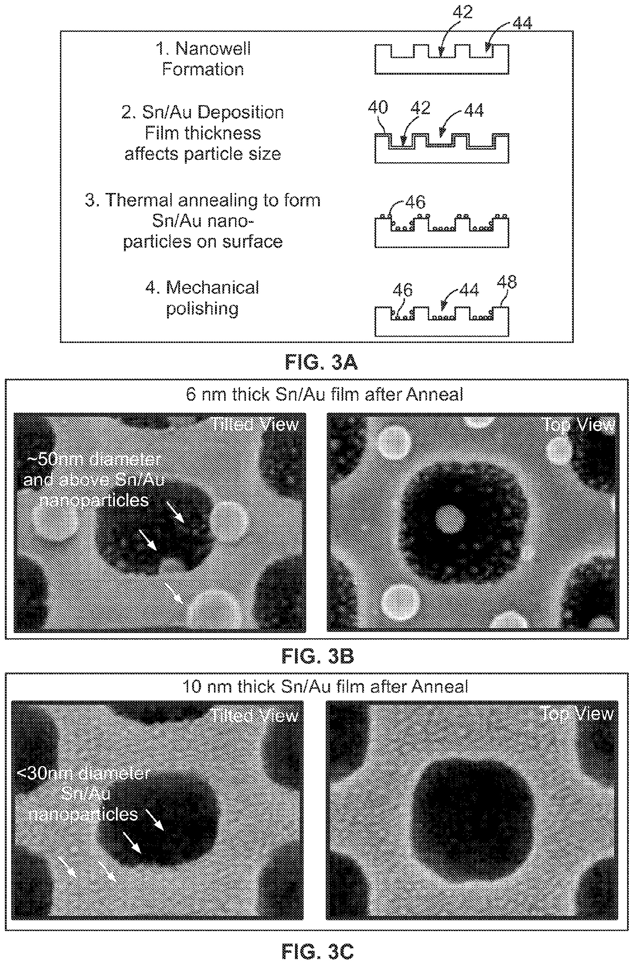

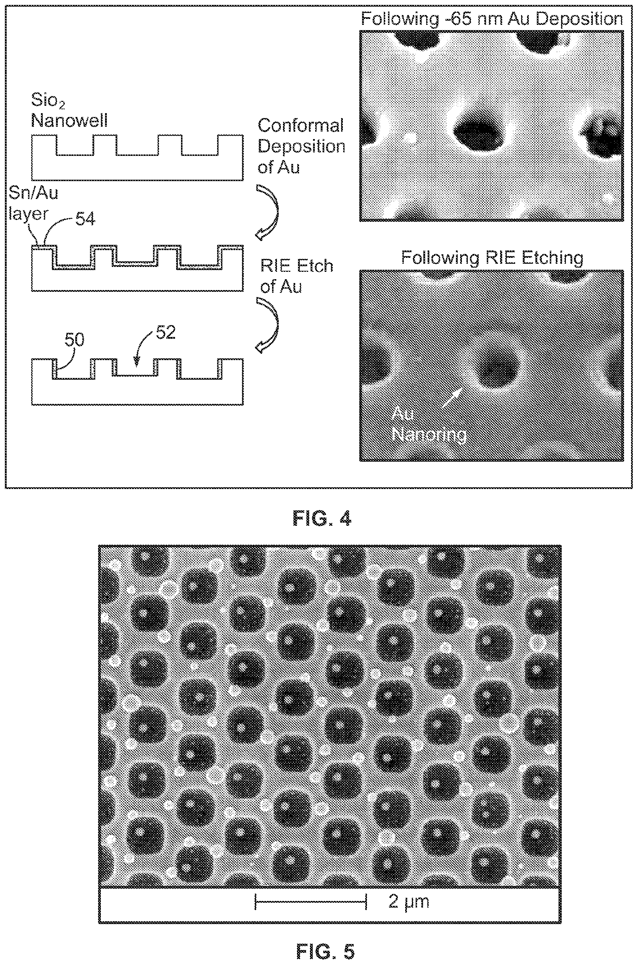

In some embodiments, the nanoparticles are formed in a well (or concave feature or reaction cavity) of a solid surface. As shown in Figure 3A, a film of starting material 40 such as Sn/Au can be deposited on a solid surface 42 containing nanowells 44, followed by thermal annealing. In some embodiments, thermal annealing can be utilized to promote formation of nanoparticles 46 as the film coalesces into discrete particles. As demonstrated by the results shown in Figures 3B and 3C, nanoparticle size can be a function of the starting film thickness. A further polishing step following the thermal anneal can result in nanoparticles 46 only in the wells while leaving the interstitial regions 48 substantially void of nanoparticles. Figure 5 is a further example of deposition of a layer of Sn/Au followed by thermal annealing. The SEM image of nanoparticles shown in Figure 5 demonstrates that nanoparticles are seen in every nanowell. Nanoparticles in interstitial regions can be removed through, for example, chemical and/or mechanical polishing. A distribution of nanoparticle sizes is observed in each nanowell enabling broad spectrum fluorescence enhancement.

In some embodiments, a nanostructures such as a nanoring can be formed along the wall of wells (or concave features or reaction cavities) on a surface. The nanostructures can be fabricated using any one of a number of methodologies known in the art. For example, Figure 4 shows a schematic and SEM images showing formation of nanorings 50 in nanowells 52. As shown in Figure 4, Au can be deposited using sputtered deposition of a layer 54 of a material such as Au. In the embodiment shown in Figure 4, conformal deposition of a ∼65 nm Au layer was followed by a reactive ion etch (RIE) process. The remaining Au layer was located along the walls of the nanowells, forming nanorings 52 in each of the nanowells.

As used herein, the term "surface" is intended to mean an external part or external layer of a solid support or gel material. The surface can be in contact with another material such as a gas, liquid, gel, polymer, organic polymer, second surface of a similar or different material, metal, or coat. The surface, or regions thereof, can be substantially flat. The surface can have surface features such as wells, pits, channels, ridges, raised regions, pegs, posts or the like.

As used herein, the term "solid support" refers to a rigid substrate that is insoluble in aqueous liquid. The solid support may also be referred to as a substrate body. The substrate of the solid support can be non-porous or porous. The substrate can optionally be capable of taking up a liquid (e.g. due to porosity) but will typically be sufficiently rigid that the substrate does not swell substantially when taking up the liquid and does not contract substantially when the liquid is removed by drying. A nonporous solid support is generally impermeable to liquids or gases. Solid supports can optionally be inert to a chemistry that is used to modify a gel. For example, a solid support can be inert to chemistry used to attach analytes, such as nucleic acids, to gels in a method set forth herein. Exemplary solid supports include, but are not limited to, glass and modified or functionalized glass, plastics (including acrylics, polystyrene and copolymers of styrene and other materials, polypropylene, polyethylene, polybutylene, polyurethanes, Teflon™, cyclic olefins, polyimides etc.), nylon, ceramics, resins, Zeonor, silica or silica-based materials including silicon and modified silicon, carbon, metals, inorganic glasses, optical fiber bundles, and polymers.

Particular embodiments of the methods and compositions presented herein utilize a solid support having a patterned or structured substrate. The patterned or structured substrate can comprise a patterned gel array, as described in U.S. Ser. No. 13/787,396 (U.S. Patent Appl. Publ. 2014/0243224 A1). In particular embodiments, a structured substrate can be made by patterning a solid support material with wells (e.g. microwells or nanowells), coating the patterned support with a gel material (e.g. PAZAM, SFA or chemically modified variants thereof, such as the azidolyzed version of SFA (azido-SFA)) and polishing the gel coated support, for example via chemical or mechanical polishing, thereby retaining gel in the wells but removing or inactivating substantially all of the gel from the interstitial regions on the surface of the structured substrate between the wells. Primer nucleic acids can be attached to gel material. A solution of target nucleic acids (e.g. a fragmented human genome) can then be contacted with the polished substrate such that individual target nucleic acids will seed individual wells via interactions with primers attached to the gel material; however, the target nucleic acids will not occupy the interstitial regions due to absence or inactivity of the gel material. Amplification of the target nucleic acids will be confined to the wells since absence or inactivity of gel in the interstitial regions prevents outward migration of the growing nucleic acid colony. The process is conveniently manufacturable, being scalable and utilizing conventional micro- or nano-fabrication methods.

A solid support used in a structured substrate set forth herein can be made from any of a variety of materials set forth herein, for example, above in the definitions, below in the examples or immediately following. A particularly useful material is glass. Other suitable substrate materials may include polymeric materials, plastics, silicon, quartz (fused silica), borofloat glass, silica, silica-based materials, carbon, metals, an optical fiber or optical fiber bundles, sapphire, or plastic materials such as COCs and epoxies. The particular material can be selected based on properties desired for a particular use. For example, materials that are transparent to a desired wavelength of radiation are useful for analytical techniques that will utilize radiation of the desired wavelength, such as one or more of the techniques set forth herein. Conversely, it may be desirable to select a material that does not pass radiation of a certain wavelength (e.g. being opaque, absorptive or reflective). This can be useful for formation of a mask to be used during manufacture of the structured substrate, such as a method set forth herein; or to be used for a chemical reaction or analytical detection carried out using the structured substrate, such as those set forth herein. Other properties of a material that can be exploited are inertness or reactivity to certain reagents used in a downstream process, such as those set forth herein; or ease of manipulation or low cost during a manufacturing process manufacture, such as those set forth herein. Further examples of materials that can be used in the structured substrates or methods of the present disclosure are described in US Ser. No. 13/661,524 and US Pat. App. Pub. No. 2012/0316086 A1.

Particularly useful solid supports for some embodiments are located within a flow cell apparatus. Exemplary flow cells, methods for their manufacture and methods for their use are described in US Pat. App. Publ. Nos. 2010/0111768 A1 or 2012-0270305 A1; or WO 05/065814. Flow cells provide a convenient format for housing an array that is produced by the methods of the present disclosure and that is subjected to a sequencing-by-synthesis (SBS) or other technique that involves repeated delivery of reagents in cycles (e.g. synthesis techniques or detection techniques having repetitive or cyclic steps). Exemplary detection methods are set forth in further detail below.

In some embodiments a flow-cell or other vessel having multiple surfaces is used. Vessels having multiple surfaces can be used such that only a single surface has gel-containing concave features (e.g. wells). Alternatively two or more surfaces present in the vessel can have gel-containing concave features. One or more surfaces of a flow cell can be selectively detected. For example, opposing surfaces in the interior of a flow cell can be selectively addressed with focused radiation using methods known in the art such as confocal techniques.

Useful confocal techniques and devices for selectively directing radiation to multiple surfaces of a vessel (e.g. a flow cell) are described, for example, in US Pat. App. Pub. No. 2009/0272914 A1 or US Pat. No. 8,039,817.

As used herein, the term "gel material" is intended to mean a semi-rigid material that is permeable to liquids and gases. Typically, gel material can swell when liquid is taken up and can contract when liquid is removed by drying. Exemplary gels include, but are not limited to those having a colloidal structure, such as agarose; polymer mesh structure, such as gelatin; or cross-linked polymer structure, such as polyacrylamide, SFA (see, for example, US Pat. App. Pub. No. 2011/0059865 A1) or PAZAM (see, for example, US Prov. Pat. App. Ser. No. 61/753,833 or U.S. Patent Appl. Publ. No. 2011/0059865 A1). Particularly useful gel material will conform to the shape of a well or other concave feature where it resides. Some useful gel materials can both (a) conform to the shape of the well or other concave feature where it resides and (b) have a volume that does not substantially exceed the volume of the well or concave feature where it resides.

As used herein, the term "interstitial region" refers to an area in a substrate or on a surface that separates other areas of the substrate or surface. For example, an interstitial region can separate one well (or concave feature) of an array from another well (or concave feature) of the array. The two regions that are separated from each other can be discrete, lacking contact with each other. In another example, an interstitial region can separate a first portion of a feature from a second portion of a feature. In many embodiments the interstitial region is continuous whereas the features are discrete, for example, as is the case for an array of wells in an otherwise continuous surface. The separation provided by an interstitial region can be partial or full separation. Interstitial regions will typically have a surface material that differs from the surface material of the features on the surface. For example, features of an array can have an amount or concentration of gel material or analytes that exceeds the amount or concentration present at the interstitial regions. In some embodiments the gel material or analytes may not be present at the interstitial regions.

In many embodiments, the interstitial region can be substantially devoid of nanostructures by polishing the solid support, for example via chemical or mechanical polishing, thereby retaining nanostructures in the wells but removing or inactivating substantially all of the nanostructures from the interstitial regions on the surface of the structured substrate between the wells. Mechanical polishing can be carried out by applying abrasive forces to the surface of the solid support. Exemplary methods include abrasion with a slurry of beads, wiping with a sheet or cloth, scraping or the like. It will be understood that beads used for polishing or other uses set forth herein can be, but need not be, spherical. Rather beads can have irregular shapes, polygonal shapes, ovoid shapes, elongated shapes, cylindrical shapes etc. The surface of the beads can be smooth or rough. Any of a variety of particles can be useful as beads for the methods and compositions set forth herein. One example of polishing includes using a lintless (cleanroom grade) wipe coated with a 3 µm silica bead slurry (10%w/v in water) to remove interstitial nanostructures. A polishing wheel/grinder can also be used with this slurry. Mechanical polishing can also be achieved using a fluid jet or gas (e.g. air or inert gas such as Argon or Nitrogen) jet to remove gel from interstitial regions.

As used herein, the term "library," when used in reference to analytes, refers to a collection of analytes having different chemical compositions. Typically, the analytes in a library will be different species having a common feature or characteristic of a genera or class, but otherwise differing in some way. For example, a library can include nucleic acid species that differ in nucleotide sequence, but that are similar with respect to having a sugar-phosphate backbone.

As used herein, the terms "nucleic acid" and "nucleotide" are intended to be consistent with their use in the art and to include naturally occurring species or functional analogs thereof. Particularly useful functional analogs of nucleic acids are capable of hybridizing to a nucleic acid in a sequence specific fashion or capable of being used as a template for replication of a particular nucleotide sequence. Naturally occurring nucleic acids generally have a backbone containing phosphodiester bonds. An analog structure can have an alternate backbone linkage including any of a variety of those known in the art. Naturally occurring nucleic acids generally have a deoxyribose sugar (e.g. found in deoxyribonucleic acid (DNA)) or a ribose sugar (e.g. found in ribonucleic acid (RNA)). A nucleic acid can contain nucleotides having any of a variety of analogs of these sugar moieties that are known in the art. A nucleic acid can include native or non-native nucleotides. In this regard, a native deoxyribonucleic acid can have one or more bases selected from the group consisting of adenine, thymine, cytosine or guanine and a ribonucleic acid can have one or more bases selected from the group consisting of uracil, adenine, cytosine or guanine. Useful non-native bases that can be included in a nucleic acid or nucleotide are known in the art. The terms "probe" or "target," when used in reference to a nucleic acid, are intended as semantic identifiers for the nucleic acid in the context of a method or composition set forth herein and does not necessarily limit the structure or function of the nucleic acid beyond what is otherwise explicitly indicated. The terms "probe" and "target" can be similarly applied to other analytes such as proteins, small molecules, cells or the like.

As used herein, the terms "coat" and "coating" and like terms, when used as a verb, are intended to mean providing a layer or covering on a surface. At least a portion of the surface can be provided with a layer or cover. In some cases the entire surface can be provided with a layer or cover. In alternative cases only a portion of the surface will be provided with a layer or covering. The term "coat," when used to describe the relationship between a surface and a material, is intended to mean that the material is present as a layer or cover on the surface. The material can seal the surface, for example, preventing contact of liquid or gas with the surface. However, the material need not form a seal. For example, the material can be porous to liquid, gas, or one or more components carried in a liquid or gas. Exemplary materials that can coat a surface include, but are not limited to, a gel, polymer, organic polymer, liquid, metal, a second surface, plastic, silica, or gas.

Structured substrates of the present disclosure that contain nucleic acid arrays can be used for any of a variety of purposes. A particularly desirable use for the nucleic acids is to serve as capture probes that hybridize to target nucleic acids having complementary sequences. The target nucleic acids once hybridized to the capture probes can be detected, for example, via a label recruited to the capture probe. Methods for detection of target nucleic acids via hybridization to capture probes are known in the art and include, for example, those described in US Pat. Nos.7,582,420; 6,890,741; 6,913,884 or 6,355,431 or US Pat. App. Pub. Nos. 2005/0053980 A1; 2009/0186349 A1 or 2005/0181440 A1. For example, a label can be recruited to a capture probe by virtue of hybridization of the capture probe to a target probe that bears the label. In another example, a label can be recruited to a capture probe by hybridizing a target probe to the capture probe such that the capture probe can be extended by ligation to a labeled oligonucleotide (e.g. via ligase activity) or by addition of a labeled nucleotide (e.g. via polymerase activity).

A nucleic acid array can also be used in a sequencing procedure, such as a sequencing-by-synthesis (SBS) technique. Briefly, SBS can be initiated by contacting the target nucleic acids with one or more labeled nucleotides, DNA polymerase, etc. Those features where a primer is extended using the target nucleic acid as template will incorporate a labeled nucleotide that can be detected. Optionally, the labeled nucleotides can further include a reversible termination property that terminates further primer extension once a nucleotide has been added to a primer. For example, a nucleotide analog having a reversible terminator moiety can be added to a primer such that subsequent extension cannot occur until a deblocking agent is delivered to remove the moiety. Thus, for embodiments that use reversible termination, a deblocking reagent can be delivered to the flow cell (before or after detection occurs). Washes can be carried out between the various delivery steps. The cycle can then be repeated n times to extend the primer by n nucleotides, thereby detecting a sequence of length n. Exemplary SBS procedures, fluidic systems and detection platforms that can be readily adapted for use with an array produced by the methods of the present disclosure are described, for example, in Bentley et al., Nature 456:53-59 (2008), WO 04/018497; WO 91/06678; WO 07/123744; US Pat. Nos. 7,057,026; 7,329,492; 7,211,414; 7,315,019 or 7,405,281, and US Pat. App. Pub. No. 2008/0108082 A1.

Other sequencing procedures that use cyclic reactions can be used, such as pyrosequencing. Pyrosequencing detects the release of inorganic pyrophosphate (PPi) as particular nucleotides are incorporated into a nascent nucleic acid strand (Ronaghi, et al., Analytical Biochemistry 242(1), 84-9 (1996); Ronaghi, Genome Res. 11(1), 3-11 (2001); Ronaghi et al. Science 281(5375), 363 (1998); US Pat. Nos. 6,210,891; 6,258,568 and 6,274,320). In pyrosequencing, released PPi can be detected by being converted to adenosine triphosphate (ATP) by ATP sulfurylase, and the resulting ATP can be detected via luciferase-produced photons. Thus, the sequencing reaction can be monitored via a luminescence detection system. Excitation radiation sources used for fluorescence based detection systems are not necessary for pyrosequencing procedures. Useful fluidic systems, detectors and procedures that can be used for application of pyrosequencing to arrays of the present disclosure are described, for example, in WIPO Pat. App. Ser. No. PCT/US11/57111, US Pat. App. Pub. No. 2005/0191698 A1, US Pat. No. 7,595,883, and US Pat. No. 7,244,559.

Sequencing-by-ligation reactions are also useful including, for example, those described in Shendure et al. Science 309:1728-1732 (2005); US Pat. No. 5,599,675; and US Pat. No. 5,750,341. Some embodiments can include sequencing-by-hybridization procedures as described, for example, in Bains et al., Journal of Theoretical Biology 135(3), 303-7 (1988); Drmanac et al., Nature Biotechnology 16, 54-58 (1998); Fodor et al., Science 251(4995), 767-773 (1995); and WO 1989/10977. In both sequencing-by-ligation and sequencing-by-hybridization procedures, nucleic acids that are present in gel-containing wells (or other concave features) are subjected to repeated cycles of oligonucleotide delivery and detection. Fluidic systems for SBS methods as set forth herein, or in references cited herein, can be readily adapted for delivery of reagents for sequencing-by-ligation or sequencing-by-hybridization procedures. Typically, the oligonucleotides are fluorescently labeled and can be detected using fluorescence detectors similar to those described with regard to SBS procedures herein or in references cited herein.

Some embodiments can utilize methods involving the real-time monitoring of DNA polymerase activity. For example, nucleotide incorporations can be detected through fluorescence resonance energy transfer (FRET) interactions between a fluorophore-bearing polymerase and γ-phosphate-labeled nucleotides, or with zeromode waveguides. Techniques and reagents for FRET-based sequencing are described, for example, in Levene et al. Science 299, 682-686 (2003); Lundquist et al. Opt. Lett. 33, 1026-1028 (2008); Korlach et al. Proc. Natl. Acad. Sci. USA 105, 1176-1181 (2008).

Another useful application for an array of the present disclosure is gene expression analysis. Gene expression can be detected or quantified using RNA sequencing techniques, such as those, referred to as digital RNA sequencing. RNA sequencing techniques can be carried out using sequencing methodologies known in the art such as those set forth above. Gene expression can also be detected or quantified using hybridization techniques carried out by direct hybridization to an array or using a multiplex assay, the products of which are detected on an array. An array of the present disclosure can also be used to determine genotypes for a genomic DNA sample from one or more individual. Exemplary methods for array-based expression and genotyping analysis that can be carried out on an array of the present disclosure are described in US Pat. Nos.7,582,420; 6,890,741; 6,913,884 or 6,355,431 or US Pat. App. Pub. Nos. 2005/0053980 A1; 2009/0186349 A1 or 2005/0181440 A1.

Several applications for arrays of the present disclosure have been exemplified above in the context of ensemble detection, wherein multiple copies of a target nucleic acid are present at each feature and are detected together. In alternative embodiments, a single nucleic acid, whether a target nucleic acid or amplicon thereof, can be detected at each feature. For example, a gel-containing well (or other concave feature) can be configured to contain a single nucleic acid molecule having a target nucleotide sequence that is to be detected. Any of a variety of single molecule detection techniques can be used including, for example, modifications of the ensemble detection techniques set forth above to detect the sites at increased resolution or using more sensitive labels. Other examples of single molecule detection methods that can be used are set forth in US Pat. App. Pub. No. 2011/0312529 A1;
US Ser. No. 61/578,684; and US Ser. No. 61/540,714.

As used herein, the term "well" refers to a discrete concave feature in a solid support having a surface opening that is completely surrounded by interstitial region(s) of the surface. Wells can have any of a variety of shapes at their opening in a surface including but not limited to round, elliptical, square, polygonal, star shaped (with any number of vertices) etc. The cross section of a well taken orthogonally with the surface can be curved, square, polygonal, hyperbolic, conical, angular, etc.

As used herein, the term "concave feature," when used in reference to a solid support, refers to a recess or indentation in the solid support. Exemplary concave features include, but are not limited to, a well, pit, hole, depression, channel, or trough. A concave feature can optionally have a curved cross section (in the dimension orthogonal to the surface of the solid support); however, a cross section with one or more linear sections, angles or corners is also possible. Cross sections with combinations of curved and linear sections are also possible. Generally, a concave feature need not pass completely through the solid support, for example, instead having a bottom surface or point in the substrate.

The embodiments set forth below and recited in the claims can be understood in view of the above definitions.

### EXAMPLE 1

### Localized SPR on Unpatterned Surfaces

This example describes the use of localized surface plasmon resonance (SPR) to increase the fluorescence intensity of amplified DNA on a solid surface (DNA "clusters"). In this example, results are shown on unpatterned glass surface coated with silane-free acrylamide (SFA) that was spiked with Au nanoparticles. DNA clusters were then amplified on this surface.

An example workflow is presented in Figure 1. In the first panel shown, SFA spiked with Au nanoparticles 10 is coated on a glass surface 14 and amplification primers (not shown) are grafted to the SFA. In the second panel shown, template DNA 18 is seeded onto the surface or gel material. Seeded template DNA 18 is then amplified on the surface as shown in the third panel, forming clusters 20 of amplified DNA, with some clusters formed directly over Au nanoparticles 10, while other clusters are formed on a portion of the surface that does not contain Au nanoparticles. As shown in the fourth panel, fluorescence 22 from each cluster is detected during a sequencing by synthesis reaction. Proximity to Au nanoparticles results in enhanced fluorescence compared to the signal emitted from clusters that are not in proximity to Au nanoparticles.

The workflow described in Figure 1 was carried out on a D263 glass flow cell surface with 8 lanes. Lanes 1, 2 were control lanes (no nanoparticles). Lanes 3-6 were spiked with a 50 nM concentration of 6nm spherical Au nanoparticles. The high Au NP concentration prevented polymerization of the in-situ coated SFA in these lanes, preventing grafting. Lanes 7, 8 were coated with SFA spiked with 10 nM concentration Au nanoparticles. Higher fluorescence intensities were seen from Lanes 7, 8 relative to the control lanes. Sequencing results indicated that the intensity for base A increased ∼ 27% relative to the control lanes. A -24% increase in intensity was observed on average for all 4 bases relative to the control lanes.

These data demonstrate that clusters in the vicinity of Au nanoparticles exhibit enhanced fluorescence due to interaction of incident light with the underlying Au nanoparticles, inducing localized surface plasmons. Clusters not in the vicinity of nanoparticles did not exhibit this enhanced fluorescence.

### EXAMPLE 2

### Enhanced Fluorescence on Patterned Substrates with Nanoparticles

Plasmonic nanostructures can also be combined with patterned nanowell substrates for enhanced fluorescence. In this example, fluorescence enhancement was achieved using nanoparticles in nanowells. Nanoparticles were formed in nanowells using a novel Sn/Au reflow approach. As illustrated in Figure 3A, a uniform film of Sn/Au was first coated on the nanowell substrate followed by a thermal annealing process at 400°C which resulted in the reflow of the film into small nanoparticles (see Figures 3B and 3C). By controlling initial film thickness, nanoparticle size was manipulated. Examples of this are shown in Figures 3B and 3C for starting film thicknesses of 6 nm and 10 nm, respectively, resulting in >50 nm diameter particles and <30 nm particles respectively.

Following nanoparticle formation, mechanical polishing was used to remove nanoparticles from the interstitial regions resulting in only particles in wells. One advantage of this technique is loading efficiency resulting from the fact that nanoparticles are loaded into every well. A further advantage of this technique is that the broad distribution of nanoparticle sizes gives broad spectrum fluorescence enhancement compared to enhancement at a small range of wavelengths as typically achieved using a single nanoparticle size.

The above experiments were repeated 3 times on 3 identical flowcells. In 3 separate experiments, fluorescence enhancement was seen for all 4 bases in the ∼ 6nm thick Sn/Au region relative to the control D263, corresponding to nanoparticles of diameter ∼ 50 nm and above in nanowells. On average, base intensity increased -37% relative to the control in these experiments with C, G, T undergoing strong enhancement but A experiencing weaker enhancement. Fluorescence enhancement was not seen on the -10 nm thick Sn/Au region, corresponding to smaller <30 nm nanoparticles.

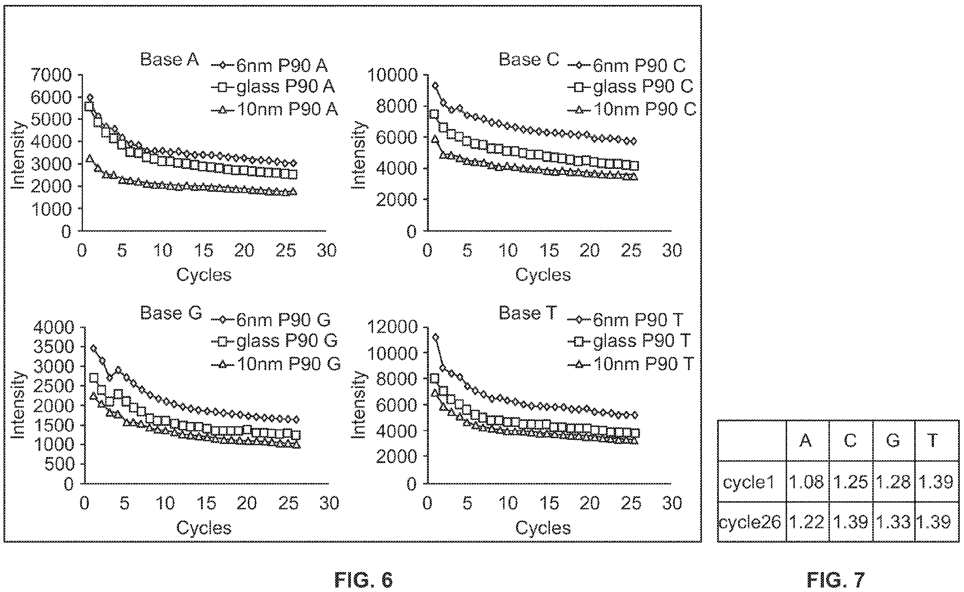

Figures 6 and 7 set forth exemplary results. Figure 6 sets forth sequencing results using isothermal amplification followed by SBS sequencing. Sequencing by synthesis was conducted for 26 cycles and compared with a control region that was void of Sn/Au deposition. Cluster intensity per cycle for each of four fluorescently labeled bases was measured as a function of Sn/Au particle thickness. The patterned substrates had -500 nm diameter wells with a pitch of 850 nm. Different Sn/Au thicknesses were deposited on different regions of a single flowcell and then annealed. Combining different film thicknesses and a control region in each lane allowed for a direct comparison of intensities from different regions of the same flow cell, thereby decoupling chemistry variations. Clusters were seen in all 3 regions of each lane, confirming reflowed Sn/Au nanoparticles of various size support clustering. A comparison of normalized cluster intensity enhancement (over control) in these regions at cycle 1 and cycle 26 is shown in Figure 7. In Figure 6, the top line illustrates the relationship between intensity and cycle number for 6nm particles, the middle line illustrates the relationship between intensity and cycle number for glass, and the bottom line illustrates the relationship between intensity and cycle number for 10nm particles.

These data demonstrate that deposition of nanoparticles in wells of a patterned surface provides enhancement of fluorescence intensity during a sequencing by synthesis reaction.

### EXAMPLE 3

### Enhanced Fluorescence on Patterned Substrates with Gold Nanoplugs

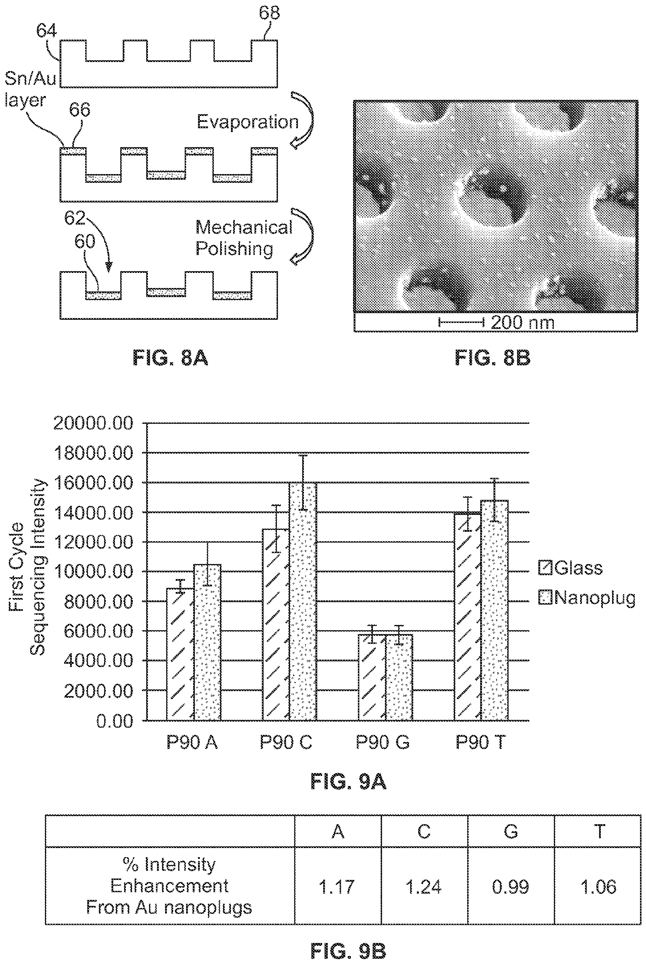

In this example, fluorescence intensity was enhanced in nanowells using gold nanoplugs deposited in the bottom of the wells. The fabrication of Au nanoplugs 60 in nanowells 62 is illustrated in Figure 8A. Briefly, Au was deposited across a patterned flow cell 64, resulting in a layer 66 of Au in the bottom of each well 62 and in each interstitial region 68. Mechanical polishing removed Au deposited on the interstitial regions 68, leaving Au plugs 60 only in the bottoms of the wells 62. Fig. 8B shows an SEM image of the resulting nanoplugs. A solid gold (Au) plug is seen at the bottom of the well with Au nanoparticles seen on the walls and in the interstitial regions. This simple structure also enables enhancement of cluster fluorescence intensity, as demonstrated by the sequencing experiment described below.

A sequencing by synthesis run was conducted on a patterned flowcell having nanoplugs in some lanes and a control region that was void of Au deposition. Cluster intensity per cycle for each of four fluorescently labeled bases was measured. Figure 9B summarizes the resulting cluster intensity enhancement for annealed Au nanoplugs in nanowells. These data demonstrate that signal intensity is enhanced by nanoplugs in wells.

Various embodiments utilize one or more nanostructures to amplify electromagnetic energy at a reaction site. For embodiments that utilize a plurality of nanostructures (e.g., two or more nanostructures), the plurality of nanostructures may be referred to as an ensemble amplifier. As used herein, the terms "nanostructure" and "nanoparticle" are used interchangeably to refer to a structure having a greatest dimension (e.g, height, width, diameter) in the range of about 1 nm to about 1000 nm, including any integer or non-integer value between 1 nm and 1000 nm. In typical embodiments, the nanoparticle is a metallic particle or a silicon particle. In some embodiments, the nanoparticle core is a spherical or nearly spherical particle of 20-200 nm in diameter. In some embodiments the range is about 1 nm to about 50 nm (for example about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 nm).

Anisotropic nanostructures (e.g., non-spherical structures) may have a length and a width or, for some embodiments, a diameter. In some embodiments, the length of the anisotropic nanostructure is the greatest dimension of the nanostructure. In some embodiments, the length of an anisotropic nanoparticle is a dimension parallel to the plane of the aperture in which the nanoparticle was produced. In some embodiments, the length of an anisotropic nanoparticle is the dimension perpendicular to the plane of the aperture in which the nanoparticle was produced. In the case of anisotropic nanostructures, the nanostructure may have a width or diameter in the range of about 50 nm to about 750 nm. In other embodiments, the nanostructure has a width or diameter of about 350 nm or less. In other embodiments, the nanoparticle has a width or diameter of 250 nm or less and in some embodiments, a width or diameter of 100 nm or less. In some embodiments, the width or diameter is between 15 nm to 300 nm.

In some embodiments, the nanoparticle has a length of about 10-750 nm. In some embodiments, the nanostructures have a preselected shape and can be, for example a nanotube, a nanowire, nanosphere, or any shape comprising the above-described dimensions (e.g., triangular, square, rectangular, or polygonal shape in 2 dimensions, or cuboid, pyramidal, cylindrical, spherical, discoid, or hemispheric shapes in the 3 dimensions). Some examples of nanostructures include, for example, bowtie nanoantennae, nanospheres, nanopyramids, nanoshells, nanorods, nanowires, nanorings, nanoplugs, nanogratings and the like. Preformed dimers and trimers of nanostructures can also be loaded into wells and have the advantage of precisely controlling nanoparticle spacing.

The nanostructures can either be fabricated on a surface or pre-formed and then loaded into reaction cavities, such as wells (e.g., nanowells). Examples of such structures include plasmonic nanoplugs fabricated at the bottom of nanowells, bowtie and cavity antennas in nanowells, metal nanogratings on which nanowells could be formed, nanostructures reflowed in nanowells or a combination of some or all of the above. One example would be a metal nanoplug in a nanowell with nanostructures on the walls formed through an electron beam evaporation process. Ensemble amplifiers or constructs (dimers, n-mers) may also be positioned within the reaction cavities. Such methods allow for precise subnanometer control over nanostructure spacings and can be formed on a large scale using bottom up self-assembly.

The spacing between any two nanostructures on a surface can be any distance. In some embodiments, the spacing can be a multiple of a wavelength of incident light energy, such as a particular emission or excitation wavelength in fluorescence spectroscopy. The spacing can be, for example, 1 λ, 2 λ, 3 λ, 4 λ or another multiple of a chosen wavelength (λ) of incident light energy. Thus, using as an example an emission wavelength (λ) of 532 nm, the spacing between nanostructures can be about 532 nm (1 λ), about 1064 nm (2 λ), or another multiple of the emission wavelength. In some embodiments, the spacing can be a fraction of a wavelength of incident light energy, such as a particular emission or excitation wavelength in fluorescence spectroscopy. The spacing can be, for example, 1 λ, ½ λ, 1/3 λ, 1/4 λ or another multiple of a chosen wavelength of incident light energy. Thus, using as an example an emission wavelength of (λ) 532 nm, the spacing between nanostructures can be about 532 nm (1 λ), 266 nm (1/2 λ), 133 nm (1/3 λ) or another fraction of the emission wavelength.

In some embodiments, the nanostructures may be referred to as "plasmonic nanostructure" or "nanoplasmonic structure." These terms may be used interchangeably and refer to any independent structure exhibiting plasmon resonance characteristic of the structure, including (but not limited to) both nanostructures, nanostructures and combinations or associations of nanostructures.

The term "nanoantenna," as used herein, includes a nanostructure or a plurality of nanostructures (or ensemble amplifier) that acts to amplify electromagnetic energy, such as light energy. As used herein, a nanoantenna (or ensemble amplifier) does not necessarily exhibit plasmon resonance characteristics. In some embodiments, a nanoantenna does not substantially comprise a plasmon resonant material. Thus, in some embodiments, nanoantennas are presented which are made of a non-metal material but which exhibits amplification characteristics of electromagnetic energy. Nanostructures presented herein can be of any suitable shape and size so as to produce the desired energy amplification. Some exemplary shapes of nanoantennas include, for example, bowtie nanoantennae, nanospheres, nanopyramids, nanoshells, nanorods, nanowires, nanorings, nanoplugs, nanogratings and the like. It will be appreciated that any of a number of known methods can be suitable for fabrication and/or deposition of nanoantenna on a solid support. Methods for fabrication of nanoantenna are known in the art and include, for example, the methods described herein for nanoparticle fabrication and deposition.

The nanostructures can comprise any material suitable for use in the methods and compositions described herein, for example, any type of material exhibiting surface plasmon resonance (SPR). In certain preferred embodiments, the nanoparticle comprises a plasmon resonant material. Examples include, but are not limited to, metal nanostructures. For example, the nanostructures can comprise a metal such as one or more of Gold (Au), Silver (Ag), Tin (Sn) Rhodium (Rh), Ruthenium (Ru), Palladium (Pd), Osmium (Os), Iridium (Ir), Platinum (Pt), Titanium (Ti) and Aluminum (Al), Chromium (Cr), Copper (Cu), or any other suitable metal. The nanostructures can be formed from a single material such as, for example a single metal. Additionally or alternatively, the nanostructures can be formed from a combination of two or more different materials, such as, for example, two or more metals. For example, the nanostructures can comprise a metal/metal mixture such as Sn/Au or Ag/Au. Alternatively or additionally, vertical layered nanostructures, such as multilayer structures of the metal-insulator-metal type may be applied. Examples include p-type doped silicon, n-type doped silicon, and gallium arsenide. In particular embodiments, the nanostructures may be formed from a polymer that is coated by a plasmon resonant material and/or a metallic material.

Formation of nanostructures on a solid support can be performed using any one of a number of methods known in the art. Nanostructures can be formed using bottom-up self-assembly of plasmonic nanostructures and nano-antennae on the sequencing substrate. For example, any one of a number of methods for deposition of layers of a material can be used, such as those described by Gaspar et al. (Scientific Reports, 2013, 3, 1469). Layer-fabricating processes that may be used to form the nanostructures include photolithography, etching, sputtering, evaporation, casting (e.g., spin coating), chemical vapor deposition, electrodeposition, epitaxy, thermal oxidation, physical vapor deposition, and the like. In some embodiments, the nanostructures may be formed using a shadow technique. In some embodiments, the nanostructures may be formed using nanolithography, such as nanoimprint lithography (NIL).

In exemplary embodiments described herein, the nanostructures can be pre-formed and mixed in a colloid-like composition with a gel material, which is deposited on a surface. Alternatively or additionally, nanostructures can be first deposited on a surface followed by deposition of a gel material over the nanostructures. In other embodiments, a gel material can be deposited on a surface and nanostructures are deposited over the gel material.

In some embodiments, the nanostructures are formed in a well (or concave feature) of a solid surface. A film of starting material such as Sn/Au can be deposited on a solid surface containing nanowells, followed by thermal annealing. In some embodiments, thermal annealing can be utilized to promote formation of nanostructures as the film coalesces into discrete particles. Nanoparticle size can be a function of the starting film thickness. A further polishing step following the thermal anneal can result in nanostructures only in the wells while leaving the interstitial regions substantially void of nanostructures. Nanostructures in interstitial regions can be removed through, for example, chemical and/or mechanical polishing. A distribution of nanoparticle sizes is observed in each nanowell enabling broad spectrum fluorescence enhancement.

In some embodiments, a nanostructures such as a nanoring can be formed along the wall of wells (or concave features) on a surface. The nanostructures can be fabricated using any one of a number of methodologies known in the art. For example, Au can be deposited using sputtered deposition. In an embodiment, conformal deposition of a ∼65 nm Au layer may be followed by a reactive ion etch (RIE) process. The remaining Au layer was located along the walls of the nanowells, forming nanorings in each of the nanowells.

The terms "excitation light" and "light emissions" mean electromagnetic energy and are used to differentiate the source of the electromagnetic energy. Excitation light is generally provided from a light source (e.g., laser) that is positioned a distance away from the reaction site. For example, for embodiments that include reaction cavities, the light source may be positioned outside of the reaction cavities. Light emissions, however, are typically generated by an emitter within or at the reaction sites. The emitter may be, for example, a fluorophore. Particular embodiments may be configured to amplify electromagnetic energy at any wavelength between 300 nm to 750 nm (e.g., 300 nm, 301 nm, 302 nm, 303 nm, 304 nm, 305 nm, 306 nm, 307 nm, 308 nm ...745 nm, 746 nm, 747 nm, 748 nm, 749 nm, and 750 nm). As used herein, the term "wavelength" shall not be limited to a single wavelength unless expressly stated to constitute "a single wavelength" or "only one wavelength". Instead, the term "wavelength" shall encompass a narrow range of wavelengths located about a desired or target wavelength (e.g., 532 nm ± 10 nm, 532 nm ± 5 nm, 660 nm ± 10 nm, 660 nm ± 5 nm).

The nanostructures of each ensemble amplifier may be configured relative to one another to amplify the electromagnetic energy in a designated manner. For example, a distance that separates the nanostructures of a corresponding ensemble amplifier may be based on the electromagnetic energy that is desired to be amplified. The nanostructures of the ensemble amplifier may be configured for a particular wavelength (e.g., narrow band of wavelengths). For example, one or more embodiments may be configured to amplify electromagnetic energy having a wavelength of 532 nm. One or more embodiments may be configured to amplify electromagnetic energy having a wavelength of 660 nm. In some embodiments, the ensemble amplifiers may be capable of amplifying multiple wavelengths or broader ranges of wavelengths.

In some embodiments, the ensemble amplifier has a polarized configuration such that a response from the reaction site is based on a polarization of the electromagnetic energy. For example, the ensemble amplifier may be configured to preferentially respond to electromagnetic energy having one or more predetermined polarizations. When the ensemble amplifier is illuminated by electromagnetic energy having a predetermined polarization, the light emissions may have a signal intensity that is larger than the signal intensity of the light emissions if the electromagnetic energy did not have the predetermined polarization. For instance, when the ensemble amplifier is illuminated by electromagnetic energy having a predetermined polarization, the light emissions may have a signal intensity that is X. In this example, the ensemble amplifier may have a dipole moment that is essentially parallel to the electromagnetic energy with the predetermined polarization. As used herein, substantially parallel may be +/- 30° from being parallel to the dipole moment. In some embodiments, substantially parallel may be +/- 25° from being parallel to the dipole moment or +/- 20° from being parallel to the dipole moment. In particular embodiments, essentially parallel may be +/- 15° from being parallel to the dipole moment or +/- 10° from being parallel to the dipole moment. In more particular embodiments, essentially parallel may be +/- 8° from being parallel to the dipole moment, +/- 5° from being parallel to the dipole moment, or +/- 3° from being parallel to the dipole moment.

When the ensemble amplifier is illuminated by electromagnetic energy that does not have the predetermined polarization, the light emissions may have a signal intensity that is 0.4X or less (40% or less) than the signal intensity when the dipole moment is essentially parallel to the electromagnetic energy. In this example, the ensemble amplifier may have a dipole moment that is essentially perpendicular to the electromagnetic energy with the predetermined polarization. As used herein, substantially perpendicular may be +/- 30° from being perpendicular (90°) to the dipole moment. In some embodiments, substantially perpendicular may be +/- 25° from being perpendicular to the dipole moment or +/- 20° from being perpendicular to the dipole moment. In some embodiments, essentially perpendicular may be +/- 15° from being perpendicular to the dipole moment or +/- 10° from being perpendicular to the dipole moment. In more particular embodiments, essentially perpendicular may be +/- 8° from being perpendicular to the dipole moment, +/- 5° from being perpendicular to the dipole moment, or +/- 3° from being perpendicular to the dipole moment.

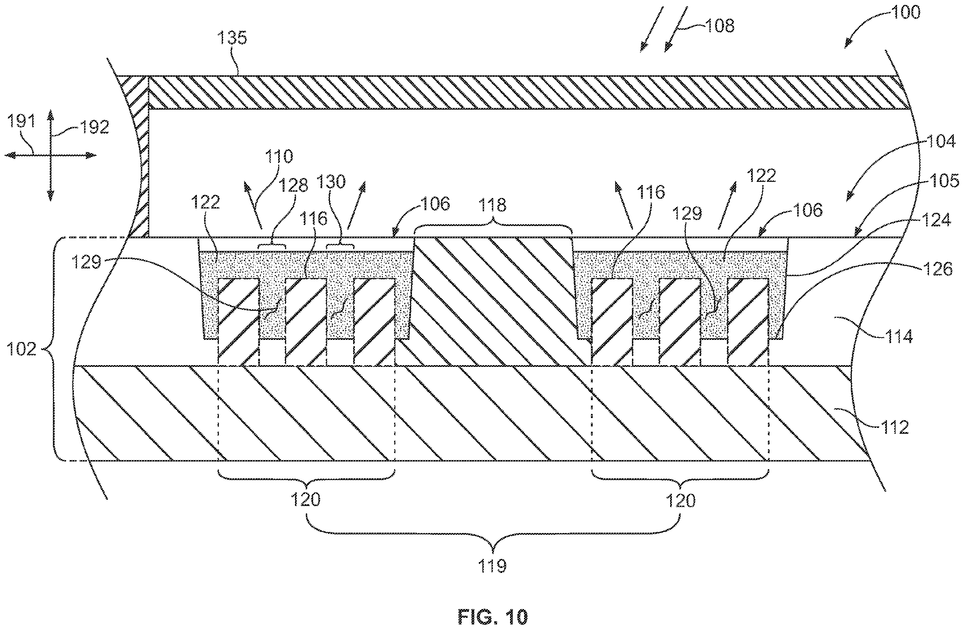

Figure 10 is a cross-section of a portion of a structured substrate 100 formed in accordance with an embodiment. The structured substrate 100 includes a substrate body 102, which may also be referred to as a solid support. The substrate body (or solid support) 102 has an active side 104. The active side 104 includes a plurality of reaction sites 106 and a side surface 105 that extends between the reaction sites 106. As shown, the reaction sites 106 are reaction cavities or wells (e.g., nanowells). Accordingly, the reaction sites 106 will be hereinafter referred to as reaction cavities 106, but it is understood that other embodiments may include reaction sites. The reaction cavities 106 may have similar dimensions the nanowells described herein. For example, the reaction cavities 106 may have a diameter or width measured along a lateral axis 191. The diameter or width of the reaction cavities may be less than 5000 nm. In some embodiments, the diameter or width of the reaction cavities is less than 4000 nm, less than 3000 nm, less than 2000 nm, or less than 1000 nm. In particular embodiments, the diameter or width of the reaction cavities is less than 900 nm, less than 800 nm, less than 700 nm, or less than 600 nm. In more particular embodiments, the diameter or width of the reaction cavities is less than 550 nm, less than 500 nm, less than 450 nm, or less than 400 nm. Yet in more particular embodiments, the diameter or width of the reaction cavities is less than 350 nm, less than 300 nm, less than 250 nm, less than 200 nm, less than 150 nm, or less than 100 nm. Reaction sites described herein may have similar diameters or widths.

The reaction cavities 106 are spaced apart from each other by interstitial regions 118 of the substrate body 102. The interstitial regions 118 are areas along the active side 104 or portions of the substrate body 102 that separate the reaction cavities 106 from one another. The side surface 105 extends along the interstitial regions 118. In some embodiments, the plurality of reaction cavities 106 form a dense array of reaction sites 106 such that adjacent reaction cavities 106 are separated, for example, by less than 1000 nm. In more particular embodiments, the adjacent reaction cavities 106 may be separated by less than 900 nm, less than 800 nm, less than 700 nm, or less than 600 nm. In more particular embodiments, the adjacent reaction cavities 106 may be separated by less than 500 nm, less than 400 nm, less than 300 nm, or less than 200 nm. In more particular embodiments, the adjacent reaction cavities 106 may be separated by less than 150 nm, less than 100 nm, or less than 50 nm. Reaction sites described herein may have similar separation distances. In some embodiments, a center-to-center spacing 119 between adjacent reaction cavities 106 may be less than 1000 nm. In more particular embodiments, the center-to-center spacing 119 may be less than 700 nm, less than 600 nm, less than 500 nm, or less than 450 nm. In more particular embodiments, the center-to-center spacing 119 may be less than 400 nm, less than 350 nm, less than 300 nm, less than 250 nm, or less than 200 nm. Reaction sites described herein may have center-to-center spacings.

In the illustrated embodiment, the interstitial regions 118 form a continuous, planar side surface 105, but the interstitial regions 118 may include non-planar surfaces in other embodiments. The interstitial regions 118 may include a surface material that differs from the material of the reaction cavities 106 and may functionally isolate the reaction cavities 106 from one another. In the illustrated embodiment, only two reaction cavities 106 are shown along the active side 104. It should be understood, however, that the reaction cavities 106 may be part of an array of reaction sites that may include hundreds, thousands, or millions of reaction cavities (or sites).

The reaction cavities 106 are typically concave features that form a depression or indentation along the active side 104. The reaction cavities 106 may be, for example, wells, pits, channels, recesses, and the like. However, it should be understood that other embodiments may include reaction sites that are not located within cavities. For example, the reaction sites may be distributed along a planar surface. Such embodiments are described in U.S. Provisional Application No. 61/920,244.

The substrate body 102 may be formed from one or more stacked layers. In the illustrated embodiment, the substrate body 102 includes a base layer 112 and a cavity layer 114. The base layer 112 may be, for example, a glass (SiO₂) wafer. The cavity layer 114 may be a polymer. The substrate body 102, however, may include other layers in alternative embodiments.

As used herein, the term "layer" is not limited to a single continuous body of material unless otherwise noted. For example, each layer may be formed form multiple sub-layers of the same or different materials. Moreover, each layer may include one or more features of different materials located therein or extending therethrough. The different layers may be formed using known layer-fabricating processes, such as photolithography, etching, sputtering, evaporation, casting (e.g., spin coating), chemical vapor deposition, electrodeposition, epitaxy, thermal oxidation, physical vapor deposition, and the like. One or more layers may also be formed using nanolithography, such as nanoimprint lithography (NIL). As used herein, the term "working substrate" includes one or more stacked layers in which at least one of the layers is being processed to form a structured substrate from the working substrate. The working substrate may form a solid support or substrate body that is configured to receive one or more other elements to form the structured substrate. For example, the working substrate may receive nanoparticles and/or an organic material (e.g., gel material) to form a structured substrate.

As shown in Figure 10, a reaction cavity has a cross section that is taken perpendicular to the active side 104. The cross-section may include curved sections, linear sections, angles, corners. Generally, a reaction cavity need not pass completely through one or more layers. For example, each of the reaction cavities 106 has at least one sidewall 124 that extends between the active side 104 and a bottom surface 126 of the reaction cavity 106. Both the sidewall 124 and the bottom surface 126 are defined by the cavity layer 114. In alternative embodiments, the base layer 112 (or other layer) may define the bottom 126 of the reaction cavity 106.

The reaction cavities 106 open to the active side 104 such that the reaction cavities 106 are accessible along the active side 104. For example, the reaction cavities 106 may be capable of receiving gel material and/or fluid along the active side 104 during manufacture of the structured substrate 100 or when the structured substrate 100 is used during analysis. The active side 104 may also receive an excitation light 108 from a light source (not shown) and/or face an optical component (not shown), such as an objective lens, that detects light emissions 110 from the reaction cavities.

Each of the reaction cavities 106 may include at least one nanostructure 116. The interstitial regions 118 may be substantially devoid of nanostructures. In the illustrated embodiment of Figure 10, each of the reaction cavities 106 includes a plurality of nanostructures 116. However, it should be understood that alternative embodiments may include only a single nanostructure. The plurality of nanostructures 116 may form an ensemble of nanostructures, which is hereinafter referred to as an ensemble amplifier 120. The ensemble amplifier 120 is positioned within each of the reaction cavities 106 and is configured to at least one of amplify electromagnetic energy that propagates into the corresponding reaction cavity or amplify electromagnetic energy that is generated within the corresponding reaction cavity.

As used herein, an "ensemble of nanostructures" or "ensemble amplifier" includes a plurality of nanostructures that are configured to at least one of amplify electromagnetic energy that is incident on the discrete site (e.g., reaction cavity) or amplify electromagnetic energy that is generated at the discrete site. For instance, the electromagnetic energy may be the excitation light 108 that propagates from an exterior environment and into a reaction cavity 106, wherein the excitation light is absorbed by an emitter (e.g., fluorophore) that is associated with a biological substance. As another example, the electromagnetic energy may be light emissions 110 that are emitted from the biological substances. More specifically, after being excited, the fluorophores may emit the electromagnetic energy (e.g., light emissions 110) that is then amplified by the ensemble 120 of nanostructures. In some embodiments, the ensemble amplifier 120 may also be referred to as a nanoantenna, because the nanostructures collectively operate to amplify and transmit the light emissions 110 away from the reaction sites.

An ensemble amplifier may include two or more nanostructures that operate in concert to amplify the electromagnetic energy. As described herein, in some embodiments, an ensemble amplifier may be configured to preferentially amplify a type of electromagnetic energy or, more specifically, electromagnetic energy having a predetermined wavelength. For example, an ensemble amplifier may have a greater amplification effect on light emissions than on excitation light or vice versa. Nonetheless, in some embodiments, an ensemble amplifier may amplify both the light emissions and the excitation light.

As used herein, when an ensemble amplifier is "configured to amplify electromagnetic energy," each of the nanostructures may have one or more qualities such that the ensemble amplifier collectively operate to amplify the electromagnetic energy. The qualities may include, for example, a material composition of the nanostructure, a shape of the nanostructure, a size of the nanostructure, and a position of the nanostructure relative to other nanostructures in the ensemble. For example, adjacent nanostructures 116 may have a distance 128 therebetween that is configured amplify electromagnetic energy that is confined therebetween. Without wishing to be bound to a particular theory, the resulting amplification in light emissions may be due to a combination of localized surface plasmon resonance and resonant energy transfer processes.

Also shown in Figure 10, the reaction cavities 106 may include an organic material 122 disposed within the reaction cavities 106. The organic material 122 may cover the nanostructures 116. In some embodiments, the organic material 122 is configured to hold or immobilize a biomolecule within the corresponding reaction cavity. For example, the biomolecule may be a nucleic acid.

In particular embodiments, the organic material 122 includes a gel material, such as a hydrogel. As used herein, the term "gel material" is intended to mean a semi-rigid material that is permeable to liquids and gases. Typically, gel material can swell when liquid is absorbed or received by the gel material and can contract when liquid is removed from the gel material (e.g., through drying). Exemplary gel materials include, but are not limited to those having a colloidal structure, such as agarose; polymer mesh structure, such as gelatin; or cross-linked polymer structure, such as polyacrylamide, SFA (see, for example, US Pat. App. Pub. No. 2011/0059865 A1) or PAZAM (see, for example, U.S. Prov. Pat. App. Ser. No. 61/753,833). Particularly useful gel material will conform to the shape of a reaction cavity where it resides. Some useful gel materials can both (a) conform to the shape of the reaction cavity where it resides and (b) have a volume that does not substantially exceed the volume of the reaction cavity where it resides.

In particular embodiments, the organic material 122 has a volume that is configured to accommodate only a single biomolecule (e.g., nucleic acid) such that steric exclusion prevents more than one biomolecule from being captured or seeding the reaction cavity. Steric exclusion can be particularly useful for nucleic acids. More specifically, reaction cavities can expose a surface of the organic material (e.g., gel material) having an area that is equivalent to or smaller than a diameter of the excluded volume of target nucleic acids that are to be seeded on the substrate. The excluded volume for a target nucleic acid and its diameter can be determined, for example, from the length of the target nucleic acid. Methods for determining the excluded volume of nucleic acids and the diameter of the excluded volume are described, for example, in U.S. Pat. No. 7,785,790; Rybenkov et al., Proc. Natl. Acad. Sci. U.S.A. 90: 5307-5311 (1993); Zimmerman et al., J. Mol. Biol. 222:599-620 (1991); or Sobel et al., Biopolymers 31:1559-1564 (1991). Conditions for steric exclusion are set forth in U.S. Ser. No. 13/661,524 and U.S. Pat. No. 7,785,790, and can be readily used for structured substrates of the present disclosure.

In some embodiments, such as embodiments that utilize steric exclusion, a library of target nucleic acids can be delivered to reaction cavities that contain the gel material prior to initiation of an amplification process. For example, target nucleic acids can be delivered to a structured substrate under conditions to seed the gel material in the substrate with the target nucleic acids. The structured substrate can optionally be washed to remove target nucleic acids that do not seed the gel material as well as any other materials that are unwanted for subsequent processing or use of the structured substrate.

Nonetheless, it will be understood that in other embodiments, the area of the exposed gel material may be substantially greater than the diameter of the excluded volume of the target nucleic acids that are transported to the amplification sites. Thus, the area for the features can be sufficiently large that steric exclusion does not occur.

Returning to Figure 10, in some embodiments, the nanostructures 116 are formed along the base layer 112 such that the nanostructures 116 project from the base layer 112 and into the reaction cavities 106. In some embodiments, the nanostructures 116 extend through a portion of the cavity layer 114. In other embodiments, the bottom surface 126 may be defined by a portion of the base layer 122 such that the nanostructures 116 do not extend through the cavity layer 114. Also shown in Figure 10, a flow cover 135 may be mounted to the substrate body 102.

During a protocol in which light emissions are detected by a detector, the light emissions may be generated in response to the excitation light 108. In alternative embodiments, the excitation light 108 is not provided and, instead, the excitation light 108 is generated by emitters coupled to the biomolecule 129. In some embodiments, a gain field 130 exists along one of the nanostructures 116 or between two or more nanostructures 116. The gain field 130 may represent a space where a high intensity electric field is created by the nanostructures 116 in response to excitation light and/or light emissions. For some applications, the nanostructures 116 amplify the excitation light 108 such that the emitters are energized by the excitation light and provide a greater signal intensity for detection. In other applications, the nanostructures 116 do not amplify the excitation light 108, but amplify the light emissions 110 such that the light emissions 110 provide a greater signal intensity for detection. In some applications, however, the nanostructures 116 may be capable of amplifying both the excitation light 108 and the light emissions 110 such that a greater intensity of the excitation light 108 is experienced by the emitters and a greater intensity of the light emissions 110 is provided by the emitters. Accordingly, embodiments set forth herein may provide a greater signal intensity that is easier to detect by imaging systems or devices compared to known systems that do not include nanostructures or ensemble amplifiers.

The present application describes various methods for manufacturing or fabricating structured substrates that may be used to detect or analyze designated reactions. At least some of the methods are illustrated in the figures as a plurality of steps. However, it should be understood that embodiments are not limited to the steps illustrated in the figures. Steps may be omitted, steps may be modified, and/or other steps may be added. By way of example, although some embodiments described herein may include only two layers, other embodiments may include three, four, or more layers. Moreover, steps described herein may be combined, steps may be performed simultaneously, steps may be performed concurrently, steps may be split into multiple sub-steps, steps may be performed in a different order, or steps (or a series of steps) may be re-performed in an iterative fashion. In addition, although different methods are set forth herein, it should be understood that the different methods (or steps of the different methods) may be combined in other embodiments.

The structured substrates may be formed using one or more processes that may, for example, be used to manufacture integrated circuits, during microfabrication, and/or to manufacture nanotechnology. Lithography (e.g., photolithography) is one category of techniques or processes that may be used to fabricate the structured substrates described herein. In particular embodiments, one or more layers are formed using nanoimprint lithography (NIL). Exemplary lithographic techniques or processes are described in greater detail in Marc J. Madou, Fundamentals of Microfabrication and Nanotechnology: Manufacturing Techniques for Microfabrication and Nanotechnology, Vol. II, 3rd Edition, Part I (pp. 2-145).

One or more processes for fabricating structured substrates may also include subtractive techniques in which material is removed from a working substrate. Such processes include chemical techniques, such as dry chemical etching, physical/chemical etching, vapor phase etching, chemical machining (CM), anisotropic wet chemical etching, wet photoetching; electrochemical techniques, such as electrochemical etching (ECM), electrochemical grinding (ECG), photoelectrochemical etching; thermal techniques, such as laser machining, electron beam machining, electrical discharge machining (EDM); and mechanical techniques, such as physical dry etching, sputter etching, ion milling, water-jet machining (WJM), abrasive water-jet machining (AWJM), abrasive jet machining (AJM), abrasive grinding, electrolytic in-process dressing (ELID) grinding, ultrasonic drilling, focused ion beam (FIB) milling, and the like. The above list is not intended to be limiting and other subtractive techniques or processes may be used. Exemplary subtractive techniques or processes are described in greater detail in Marc J. Madou, Fundamentals of Microfabrication and Nanotechnology: Manufacturing Techniques for Microfabrication and Nanotechnology, Vol. II, 3rd Edition, Part II (pp. 148-384).

One or more processes for fabricating structured substrates may also include additive techniques in which material is added to a working substrate. Such processes include physical vapor deposition (PVD), evaporation (e.g., thermal evaporation), sputtering, ion plating, ion cluster beam deposition, pulsed laser deposition, laser ablation deposition, molecular beam epitaxy, chemical vapor deposition (CVD) (e.g., atmospheric pressure CVD (APCVD), low pressure CVD (LPCVD), very low pressure CVD (VLPCVD), ultrahigh vacuum CVD (UHVCVD), metalorganic CVD (MOCVD), laser-assisted chemical vapor deposition (LCVD), plasma-enhanced CVD (PECVD), atomic layer deposition (ALD)), epitaxy (e.g., liquid-phase epitaxy, solid-phase epitaxy), anodization, thermal spray deposition, electroplating, implantation, diffusion, incorporation in the melt, thermal oxidation, laser sputter deposition, reaction injection molding (RIM), self-assembled monolayers (SAMs), sol-gel addition, spin coating, polymer spraying, polymer dry film lamination, casting, plasma polymerization, silk screening, ink jet printing, mechanical microspotting, microcontact printing, stereolithography or microphotoforming, electrochemical forming processes, electrodeposition, spray pyrolysis, laser beam deposition, electron beam deposition, plasma spray deposition, micromolding, LIGA (which is a German acronym for x-ray lithography, electrodeposition, and molding), compression molding, and the like. The above list is not intended to be limiting and other additive techniques or processes may be used. Exemplary additive techniques or processes are described in greater detail in Marc J. Madou, Fundamentals of Microfabrication and Nanotechnology: Manufacturing Techniques for Microfabrication and Nanotechnology, Vol. II, 3rd Edition, Part III (pp. 384-642). As used herein, the term "exemplary embodiment," means an embodiment that serves as one example. The term does not indicate that the embodiment is preferred over other embodiments.

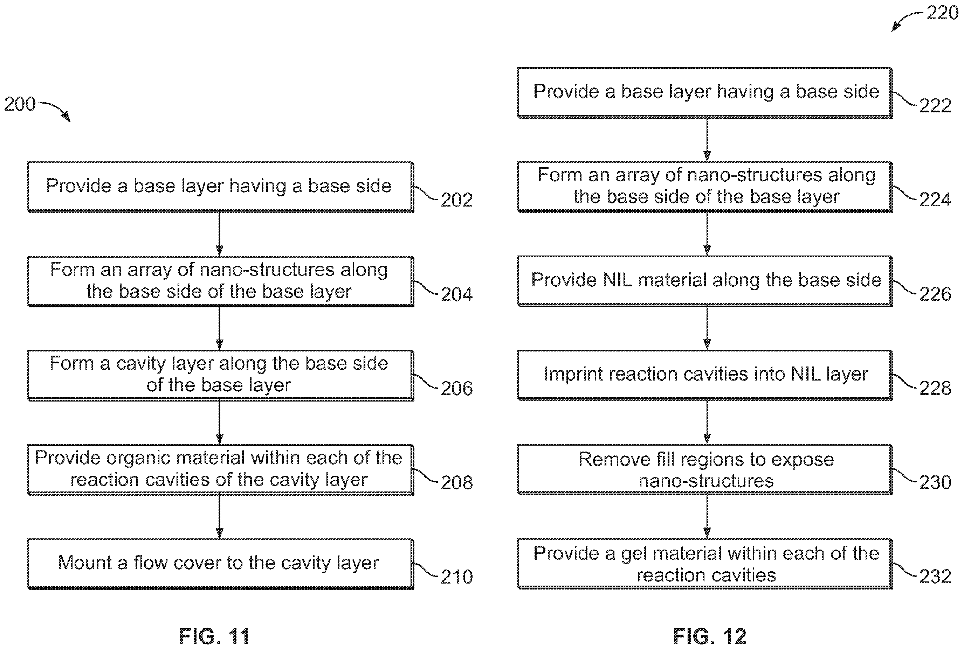

Figure 11 is a flowchart illustrating a method 200 of manufacturing a structured substrate. The method 200 includes providing, at 202, a base layer (or working substrate) having a base side. The base layer may be only a single layer of material or include one or more sub-layers. The base side may have a planar surface that is configured to have another layer deposited directly thereon. However, it is contemplated that the base side may include non-planar features prior to being combined with other layers. In particular embodiments, the base layer includes a glass (SiO₂) wafer, but other materials may be used.

The method 200 may also include forming, at 204, an array of nanostructures along the base side of the base layer. The forming, at 204, may include multiple processing steps. For example, the forming, at 204, may include providing (e.g., through deposition, growing, or another additive technique) a feature layer along the base side of the base layer. The forming, at 204, may include shaping (e.g., through etching or another subtractive technique) a sub-layer of the base layer to form the nanostructures. The sub-layer may also be referred to as a feature layer as the nanostructures may be formed from the sub-layer. The feature layer may include a material that is capable of being shaped into individual features that may at least partially form a basis of the nanostructures. The material may include a pure material (e.g., gold) or an alloy of material. The feature layer may also include multiple sub-layers of material (e.g., gold and chrome) that are stacked alongside each other. Optionally, one or more of the materials is a plasmon resonant material.

In particular embodiments, the forming, at 204, includes etching the feature layer to form nano-bodies. The nano-bodies may be arranged in sub-arrays or sets in which each sub-array (or set) may become an ensemble amplifier. In some embodiments, the nano-bodies formed from the etching process may constitute, without further modification, nanostructures that are capable amplifying electromagnetic energy. In other embodiments, however, further processing steps may be necessary to form the nanostructures. For example, the feature layer may comprise a polymer (or other material that is not a plasmon resonant material) that may be shaped to form nano-bodies for constructing the nanostructures. A thin layer or film may be subsequently added to exterior surfaces of the nano-bodies to form the nanostructures. Yet still in other embodiments, the nanostructures may be locally deposited at select locations.

In some embodiments, the nanostructures may be similar to and/or formed in a similar manner as described in: Li, Zhipeng, et al. "Multiple-particle nanoantennas for enormous enhancement and polarization control of light emission." Acs Nano 3.3 (2009): 637-642; Bharadwaj, Palash, et al. "Optical Antennas" Advances in Optics and Photonics 1, 438-483 (2009); Boltasseva, Alexandra. "Plasmonic components fabrication via nanoimprint." Journal of Optics A: Pure and Applied Optics 11.11 (2009): Kinkhabwala, Anika, et al. "Large single-molecule fluorescence enhancements produced by a bowtie nanoantenna." Nature Photonics 3.11 (2009): 654-657; Bakker, Reuben M., et al. "Nanoantenna array-induced fluorescence enhancement and reduced lifetimes." New Journal of Physics 10.12 (2008); Liang, Chia-Ching, et al. "Plasmonic metallic nanostructures by direct nanoimprinting of gold nanoparticles." Optics express 19.5 (2011): 4768-4776; Olmon, Robert L., and Markus B. Raschke. "Antenna-load interactions at optical frequencies: impedance matching to quantum systems." Nanotechnology 23.44 (2012); Krasnok, Aleksandr E., et al. "Optical nanoantennas." Physics-Uspekhi 56.6 (2013)..

The method 200 also includes forming, at 206, a cavity layer along the base side of the base layer. The cavity layer is configured to include the reaction cavities. For embodiments that do not include reaction cavities, the cavity layer may be referred to as a site layer. As used herein, the phrase "along the base side" or "along the base layer" includes the cavity layer being in direct contact with the base layer or includes the cavity layer being separated from the base layer by one or more intervening layers. As used herein, spatially relative terms, such as "top," "above," "below," and the like, are used herein for ease of description to distinguish one element or feature from another. The spatially relative terms do not require that the structured substrate have a particular orientation with respect to gravity during use or operation. For example, the active side of the structured substrate may face in a direction that is opposite the gravitational force direction in some embodiments. Alternatively, the active side of the structured substrate may face in the same direction as the gravitational force direction in other embodiments. The uppermost surface, such as the side surface that liquid flows along during operation, may be referred to as the top surface regardless of the orientation of the structured substrate with respect to gravity.

The forming, at 206, may include providing a cavity layer that is configured to have an array of reaction cavities. The forming, at 206, may include multiple steps. In some embodiments, the cavity layer includes pre-formed reaction cavities. Each of the reaction cavities may be aligned with a corresponding sub-array or set of nanostructures (e.g., two or more nanostructures). Optionally, the cavity layer may be etched to remove portions of the cavity layer and expose the nanostructures within the corresponding reaction cavities.

In other embodiments, the reaction cavities may be shaped while the cavity layer is positioned above and coupled to the base layer. For example, NIL material may be deposited along the base side of the base layer after the nanostructures are formed and cover the nanostructures. The NIL material may be deposited using, for example, a spin coating technique or by depositing droplets along the base side. The NIL material may comprise a material that is capable of being imprinted using the NIL technique. For example, the NIL material may comprise a polymer. The NIL material may then be imprinted or stamped with a mold (also called template) having a pattern of features that form the reaction cavities in the NIL material. In some embodiments, the mold is transparent to allow ultraviolet (UV) or visible light to propagate therethrough. In such embodiments, the NIL material may comprise a photocurable polymer that is cured by the UV or visible light while the mold is pressed into the NIL material. Accordingly, the NIL material may cure (e.g., harden) to form the reaction cavities. This process may be identical or similar to step-and-flash imprint lithography (SFIL). In other embodiments, the NIL material may be cured by application of thermal energy and/or pressure. The NIL techniques and like processes are described in Marc J. Madou, Fundamentals of Microfabrication and Nanotechnology: Manufacturing Techniques for Microfabrication and Nanotechnology, Vol. II, 3rd Edition, Part I (pp. 113-116) and Lucas et al., "Nanoimprint Lithography Based Approach for the Fabrication of Large-Area, Uniformly Oriented Plasmonic Arrays" Adv. Mater. 2008, 20, 1129-1134.

Each of the reaction cavities may be aligned with a corresponding sub-array of nanostructures. The NIL material may be preferentially etched to expose the plurality of nanostructures within the corresponding reaction cavity. Regardless of the method of manufacturing, the sub-array of nanostructures may form an ensemble amplifier of the corresponding reaction cavity. The ensemble amplifier is configured to at least one of amplify electromagnetic energy propagating into the corresponding reaction cavity or amplify electromagnetic energy generated within the corresponding reaction cavity.

Optionally, the method 200 may also include providing, at 208, an organic material within the reaction cavities. The organic material may cover the nanostructures. In some embodiments, the organic material is provided across the active side, including the interstitial regions. The organic material may then be removed by polishing the active side. After the active side is polished, each of the reaction cavities may include corresponding organic material that is separated from other organic material of other reaction cavities. In particular embodiments, the organic material is a gel material, such as those described herein (e.g., PAZAM, SFA or chemically modified variants thereof, such as the azidolyzed version of SFA (azido-SFA).

The method 200 may also include additional steps, such as preparing surfaces of the structured substrate to interact with the fluids and samples of a designated protocol. As another example, the method 200 may include mounting, at 210, a flow cover to the active side of the cavity layer. The flow cover may define a flow channel between the flow cover and the active side. Embodiments that include flow covers are described in U.S. Provisional Application No. 61/914,275 and International Application No. PCT/US14/69373.

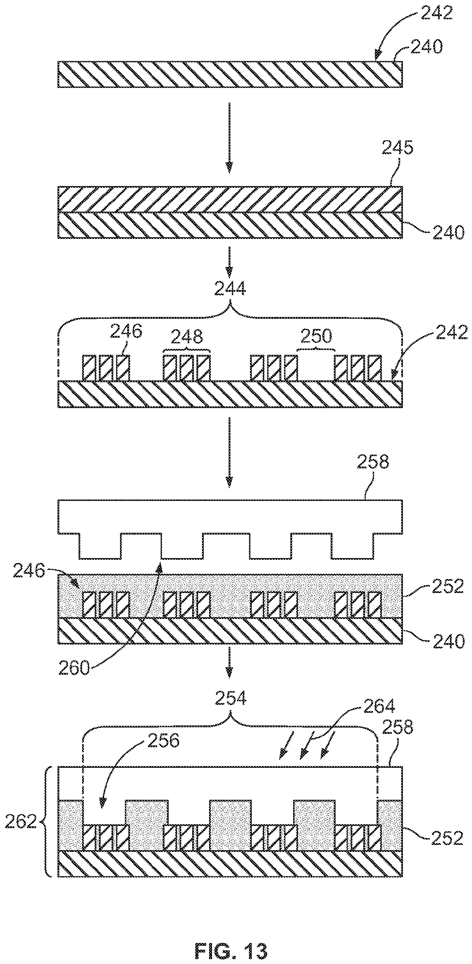

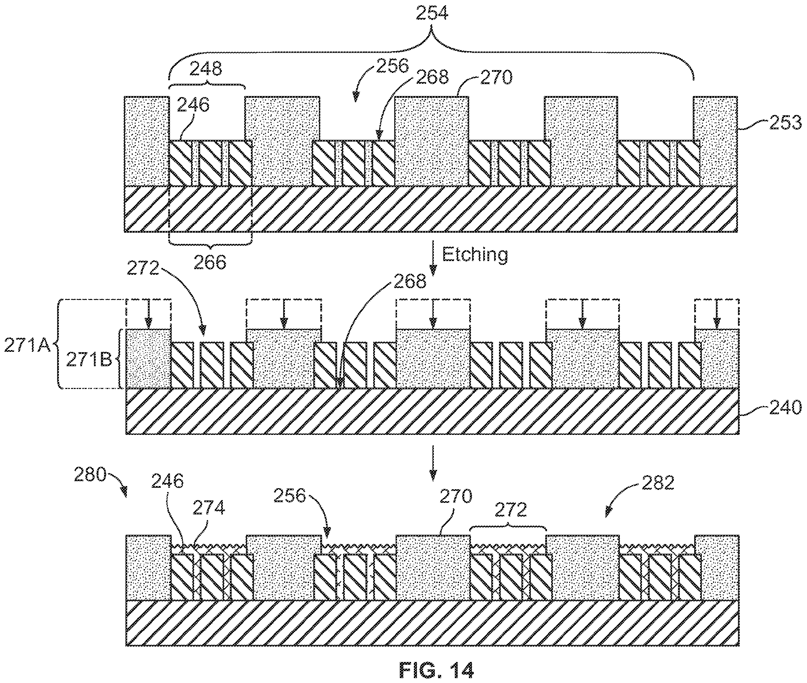

Figure 12 illustrates a flowchart of a method 220 of manufacturing a structured substrate 280 (shown in Figure 14). The method 220 is described with reference to Figures 13 and 14. The method 220 may include one or more steps that are similar or identical to the steps of method 200 (Figure 11). The method 220 includes providing, at 222, a base layer (or working substrate) 240 having a base side 242. The method 220 also includes forming, at 224, an array 244 of nanostructures 246 along the base side 242. For example, a feature layer 245 may be provided to the base side 242 (e.g., through a deposition process) of the base layer 240. The feature layer 245 may be etched to form the array 244 of nanostructures 246. The array 244 may include sub-arrays 248 of the nanostructures 246. Also shown in Figure 13, adjacent sub-arrays 248 are separated by a spacing 250 along the base side 242.

Each sub-array 248 may include a plurality of the nanostructures 246 that collectively form an ensemble amplifier when the structured substrate 280 (Figure 14) is fully formed. For example, the nanostructures 246 of each sub-array 248 may be sized, shaped, and positioned relative to each other such that the nanostructures 246 amplify electromagnetic energy. In the illustrated embodiment, the nanostructures 246 are illustrated as upright posts that have a common shape and size. However, it should be understood that the nanostructures 246 may have different shapes in other embodiments. In some embodiments, the ensemble amplifiers may have essentially the same arrangement of nanostructures 246. As used herein, "essentially the same" means that the arrangements would be identical, but for manufacturing tolerances. However, in other embodiments, the nanostructures 246 of a single sub-array 248 are not required to have an identical shape and/or an identical size.

At 226, a NIL material 252 may be provided along the base side 242 of the base layer 240. The NIL material 252 may cover the array 244 of the nanostructures 246. The NIL material 252 may be a viscous material such that the NIL material 252 surrounds and fills empty spaces between the nanostructures 246. The NIL material 252 may comprise, for example, a polymer. In the illustrated embodiment, the NIL material 252 is provided as a NIL layer along the base side 242. In other embodiments, the NIL material may be provided as an array of discrete droplets that, when compressed during an imprinting operation, effectively cover at least portions of the base side 242.

At 228, an array 254 of reaction cavities 256 may be imprinted into the NIL material 252. The imprinting, at 228, may include applying a mold 258 to the NIL material 252. The mold 258 may have a non-planar side 260 that includes a pattern of features. The features are sized, shaped, and positioned relative to each to shape the NIL material 252 in a predetermined manner such that the reaction cavities 256 are formed. When the mold 258 is applied to the NIL material 252, a stacked assembly 262 is formed that includes the mold 258, the NIL material 252, the nanostructures 246, and the base layer 240.

The imprinting, at 228, may also include curing the NIL material 252 to solidify the shape of the NIL material 252. For example, the curing process may include applying a UV light or visible light 264 to the stacked assembly 262. The NIL material 252 may comprise a photopolymer that is capable of solidifying after being exposed to the UV or visible light 264. However, alternative methods of solidifying or curing the NIL material 252 may be used. For example, thermal energy (e.g., heat) or pressure may be applied to the NIL material 252 to solidify the NIL material 252 and form the reaction cavities 256.

With respect to Figure 14, after the curing process, the NIL material becomes a solidified NIL layer 253 having the array 254 of reaction cavities 256. The solidified NIL layer 253 may constitute a cavity layer, such as the cavity layer 114 (Figure 10), that includes the reaction cavities 256. Each reaction cavity 256 may be aligned with a corresponding sub-array 248 of the nanostructures 246 such that the reaction cavity 256 is positioned above the corresponding sub-array 248. As shown in Figure 13, the nanostructures 246 may be positioned within a fill region 266 of the solidified NIL layer 253. The fill region 266 includes the nanostructures 246 surrounded by the solidified material of the NIL layer 253. At this stage, the fill region 266 may define a bottom surface 268 of the reaction cavity 256. Also shown, at this stage, the reaction cavities 256 may be separated by interstitial regions 270, which extend between the reaction cavities 256.

The method 220 may also include removing, at 230, the fill regions 266 to expose at least portions of the nanostructures 246 within the corresponding reaction cavities 256. For example, a preferential etching process may be applied to remove the material of the NIL layer 253 that surrounds the nanostructures 246 without substantially damaging or removing the nanostructures 246. During the removing, at 230, the bottom surface 268 of each reaction cavity 256 may be lowered such that the bottom surface 268 approaches the base layer 240. In some embodiments, the NIL layer 253 within the fill regions 266 may be etched entirely such that the base layer 240 forms at least a portion of the bottom surface 268. In other embodiments, similar to the structured substrate 100 of Figure 10, a portion of the NIL layer 253 may remain after the etching process. In such embodiments, the nanostructures 246 may extend through the NIL layer 253 (or cavity layer). During the removing, at 230, the interstitial regions 270 may also be etched, as indicated, such that a height of the interstitial regions 270 relative to the base layer 240 is reduced. The height is reduced from 271A to 271B.

As described above, the nanostructures 246 within each reaction cavity 256 may form an ensemble amplifier 272 of the corresponding reaction cavity 256. The ensemble amplifier 272 is configured to at least one of amplify electromagnetic energy propagating into the corresponding reaction cavity or amplify electromagnetic energy generated within the corresponding reaction cavity.

The structured substrate 280 is shown at the bottom of Figure 14. The structure substrate 280 includes an active side 282 and has the reaction cavities 256 and the interstitial regions 270 that separate the reaction cavities 256. Optionally, the method 200 may include providing, at 232, an organic material 274 within the reaction cavities 256. Prior to providing the organic material 274, the working substrate may be processed for receiving the organic material 274. For example, a passivation layer (e.g., tantalum oxide or the like) and a layer of silane may be provided onto the passivation layer. Both the passivation layer and the silane layer may cover the nanostructures 246. The providing, at 232, may include spin coating the organic material onto the working substrate. However, other additive techniques may also be used. Optionally, the working substrate having the passivation layer, the silane layer, and the organic material may be incubated.

As shown in Figure 14, the organic material 274 may cover the nanostructures 246 in the reaction cavities 256. In some embodiments, the organic material 274 is provided across the entire active side 282 such that the organic material 274 covers surfaces of the interstitial regions 270. The organic material 274 may then be removed by polishing the active side 282. After the active side 282 is polished, each of the reaction cavities 256 may include organic material 274 therein that is separated from organic material 274 in adjacent reaction cavities 256. The organic material 274 within each reaction cavity 256 surrounds the nanostructures 246 of the ensemble amplifier 272. The organic material 274 may be configured to support and/or hold a biological or chemical substance that is capable of providing light emissions, such as dye-labeled nucleic acids.

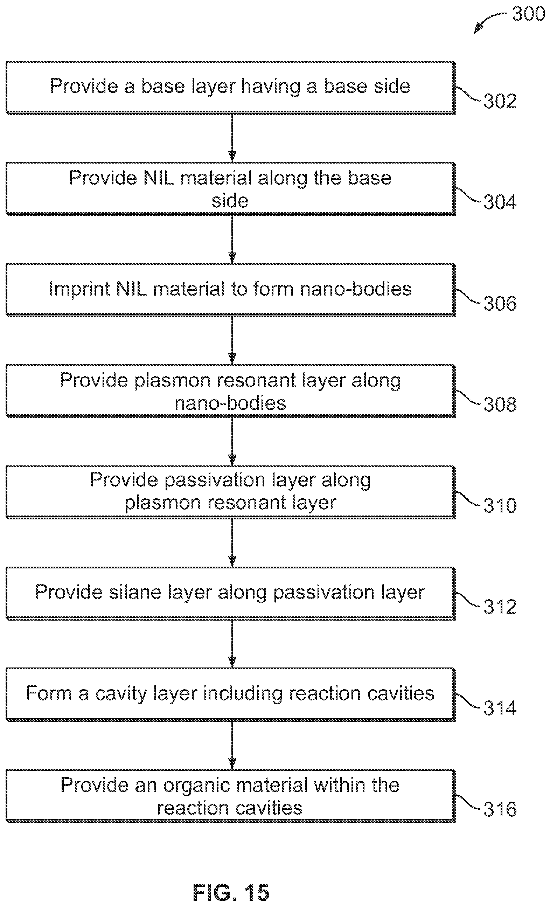

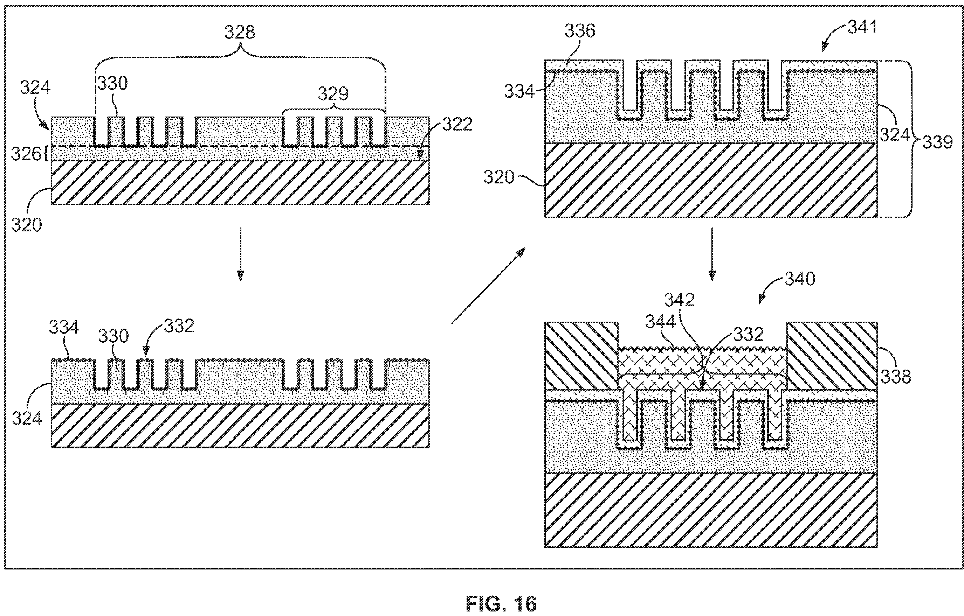

Figure 15 is a flowchart illustrating a method 300 of manufacturing or fabricating a structured substrate. In some embodiments, the method 300 includes steps that are similar or identical to the steps of the methods 200 (Figure 11) and 220 (Figure 12). Different stages of the method 300 are illustrated in Figure 16. The method 300 may include providing, at 302, a base layer (or working substrate) 320 having a base side 322 and providing, at 304, NIL material 324 along the base side 322 of the base layer 320. In some embodiments, the NIL material 324 may be provided as a NIL layer. In other embodiments, the NIL material 324 may be provided as separate droplets along the base side 322.

The method 300 may also include imprinting, at 306, the NIL material 324. After imprinting, the NIL material 324 may be a solidified NIL layer 324 having a base portion 326 (indicated by the dashed line) and an array 328 of nano-bodies 330 that project from the base portion 326. The array 328 may include multiple sub-arrays 329. The base portion 326 extends between adjacent nano-bodies 330. As such, each of the nano-bodies 330 may be part of the same patterned layer. The nano-bodies 330 may have a variety of shapes. In the illustrated embodiment, the nano-bodies 330 are elongated posts that project away from the base portion 326 of the NIL layer 324. In alternative embodiments, the base portion 326 is not formed after imprinting. Instead, only the nano-bodies 330 may be formed after imprinting. Optionally, the base portion 326 may be etched.

The method 300 may also include providing, at 308, a plasmon resonant layer 334 along the NIL material 324 and, in particular, the nano-bodies 330. The providing, at 308, may also be referred to as depositing or growing. In some embodiments, the plasmon resonant layer 334 may be a thin film or coating. The providing, at 308, may be executed using one or more additive techniques. For example, the providing, at 308, may include at least one of PECVD, ALD, evaporation, sputtering, spin coating, or the like. The plasmon resonant layer 334 includes a plasmon resonant material (e.g., gold, silver, silicon, and the like) that covers the nano-bodies 330. The plasmon resonant layer 334 may cover the entire NIL layer 324. In other embodiments, the plasmon resonant layer 334 may be selectively deposited over the sub-arrays 329. Accordingly, nanostructures 332 may be formed in which each nanostructure 332 includes a respective nano-body 330 and a portion of the plasmon resonant layer 334 that covers or surrounds the respective nano-body 330.

Optionally, the method 300 may include providing, at 310, a passivation layer 336. The passivation layer 336 is configured to protect the underlying layers, such as the plasmon resonant layer 334, from damage during use of the structured substrate. The passivation layer 336 may be similar to one or more of the passivation layers described in U.S. Provisional Application No. 61/914,275 and International Application No. PCT/US14/69373,.

Also optionally, the method 300 may include providing, at 312, an immobilization layer, hereinafter referred to as a silane layer (not shown). The silane layer (or immobilization layer) may be configured to facilitate coupling between an organic material and/or biological or chemical substances. By way of example, the providing, at 312, may be accomplished by vapor deposition. In some embodiments, the silane layer may be provided after other processing steps. At this stage, the base layer 320, the NIL material 324, the plasmon resonant layer 334, the passivation layer 336, and the optional silane layer may form a working substrate 339 having an operative side 341.

At 314, the method 300 may include forming a cavity layer 338 along the operative side 341 that includes a plurality of reaction cavities 340. In some embodiments, the cavity layer 338 may be formed using, for example, a NIL technique in which a NIL material is imprinted and cured to form the reaction cavities 340. However, other additive techniques may be used to provide the cavity layer 338. In Figure 16, only a single reaction cavity 340 is shown, but it should be understood that an array of reaction cavities 340 may be formed.

If the cavity layer 338 is formed using a NIL process, empty space between the nanostructures 332 may be filled with the NIL material 324. As described above with respect to the method 220, the NIL material 324 may be removed through preferential etching. Optionally, the silane layer may be provided after the cavity layer 338 and the reaction cavities 340 are formed along the working substrate 339. After the NIL material is removed, an ensemble amplifier 342 of the nanostructures 332 may be formed within the corresponding reaction cavity 340. At 316, an organic material may be provided to the reaction cavities 340.

In the illustrated embodiment, the cavity layer 338 is formed using a NIL process. However, it should be understood that the cavity layer 338 may be formed using other additive and, optionally, subtractive processes, such as those described above.

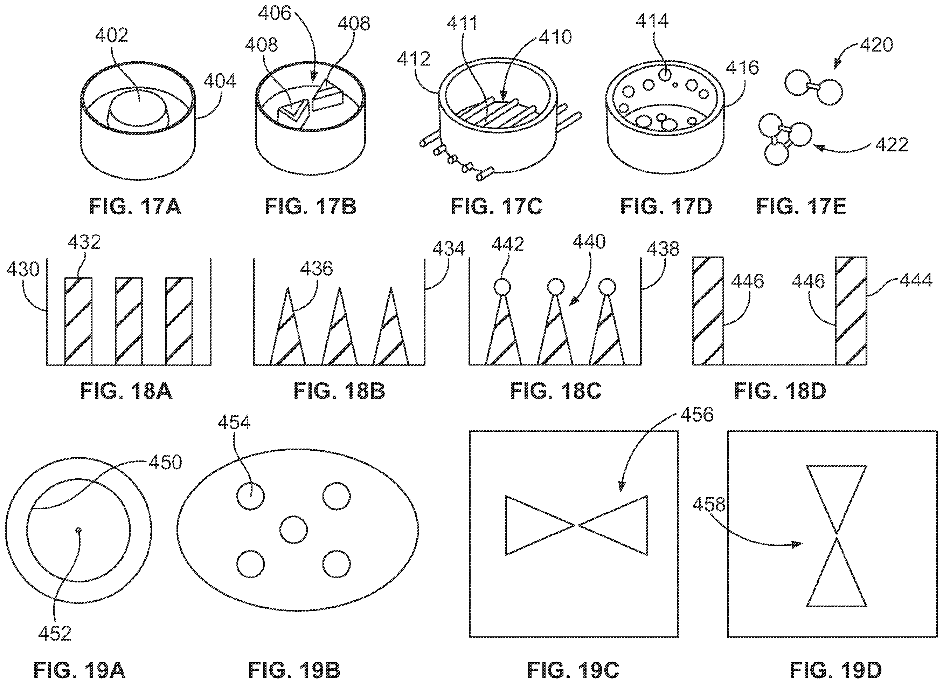

Figures 17-19 illustrate different nanostructures that may be implemented with one or more embodiments. However, the nanostructures shown in Figures 17-19 are exemplary only and are not intended to be limiting. Other nanostructures may be used in alternative embodiments. In Figures 17A-17D, the nanostructures are located within corresponding cylindrically-shaped reaction cavities. In other embodiments, the reaction cavities may have a different shape. For example, a cross-section of the reaction cavity may be oval-shaped, square-shaped, rectangular, other polygonal shape, or the like. Yet in other embodiments, the nanostructures may be located along a planar surface.

Figure 17A is a perspective view of a nanoplug 402 in a reaction cavity 404, which may also be referred to as a nanowell. The nanoplug 402 may comprise gold (Au). In the illustrated embodiment, the nanoplug 402 is centrally located within the reaction cavity 404, but it may have other positions in other embodiments. Figure 17B is a perspective view of a bowtie antenna 406 that may be use within one or more embodiments. The bowtie antenna 406 includes two separate nanostructures 408 that are triangular in shape and point to each other with a small gap therebetween. The bowtie antenna 406 may form an ensemble amplifier. Figure 17C illustrates a nanograting 410 in a reaction cavity 412 that includes a series of spaced-apart beams 411. The nanograting 410 may be formed in a lower layer and subsequently exposed when the reaction cavity 412 is formed above the nanograting 410. As shown, the nanograting 410 is not confined within the reaction cavity 412 and extends beyond the wall of the reaction cavity 412. Figure 17D illustrates a plurality of nanoparticles 414 disposed within a reaction cavity 416. The nanoparticles 414 may be distributed in random locations within the reaction cavity 416. The nanoparticles 414 may be formed, for example, through a reflow or deposition process. Figure 17E illustrates a dimer 420 and a trimer 422. The dimer 420 and the trimer 422 may be disposed within alone in a single reaction cavity (not shown) without other nanostructures disposed therein. Alternatively, the dimer 420 and trimer 422 may share a common reaction cavity. Optionally, the dimer 420 and trimer 422 are not disposed within reaction cavities and, instead, are distributed along a planar surface (not shown).

Figures 18A-18D illustrate side cross-sections of reaction cavities having nanostructures disposed therein. The reaction cavities may be, for example, cylindrical or rectangular-shaped. In Figure 18A, a reaction cavity 430 is shown that includes a plurality of nanostructures 432. The nanostructures 432 are posts that may be cylindrical or square-shaped. In Figure 18B, a reaction cavity 434 is shown that includes a plurality of nanostructures 436. The nanostructures 436 may be conical or pyramidal. In Figure 18C, a reaction cavity 438 is shown that includes a plurality of nanostructures 440. Each of the nanostructures 440 may be conical or pyramidal and have a particle portion 442 (e.g., gold particle) disposed at a top of the nanostructure 440. In Figure 18D, a reaction cavity 444 is shown that includes a plurality of nanostructures 446. The nanostructures 446 constitute sidewalls that face each other.

Figures 19A-19D illustrate plan views of reaction cavities having one or more nanostructures disposed therein. More specifically, Figure 19A illustrates a nanoring 450 that surrounds a central axis 452. The nanoring 450 is circular in Figure 19A, but may have other shapes (e.g., polygonal) in other embodiments. Figure 19B illustrates five posts 454 that are positioned relative to one another. Figures 19C and 19D show bowtie antennas 456, 458, respectively. The bowtie antennas 456, 458 are configured to preferentially respond to different polarizations of light.

In each of Figures 17A-7C, 18A-18D, and 19B-19D, the nanostructures may be configured to form a corresponding ensemble amplifier that is orientation dependent such that the ensemble amplifier preferentially responds to a polarized light of a designated orientation. Such ensemble amplifiers may be referred to as polarized amplifiers. For example, the ensemble amplifiers may be configured to have a dipole moment that is essentially parallel to an excitation light of a designated polarization. The amount of light emissions provided by reaction cavities having such polarized amplifiers is dependent upon the polarization of the excitation light.

In other embodiments, the ensemble amplifiers may be configured to preferentially respond to light emissions of a predetermined wavelength. For example, if the emitters provide light emissions that are equal to or near the predetermined wavelength, the ensemble amplifiers may amplify the light emissions. However, if the emitters provide light emissions that are not equal to or near the predetermined wavelength, the ensemble amplifiers may only partially amplify the light emissions or amplify the light emissions by a negligible amount.

Accordingly, the present application describes various embodiments and one or more aspects (e.g., features) that may be used with the embodiments. It should be understood that the various aspects may be combined and/or further modified in particular embodiments.

Various embodiments include nanostructures. In some embodiments, the nanoparticles may include dimers or trimers within the wells. In some embodiments, the nanostructures may include bowtie nanoantennae. In some embodiments, the nanostructures may include nanorods. In some embodiments, the nanostructures may include nanorings. In some embodiments, the nanostructures may include nanoplugs. In some embodiments, the nanostructures may include nanogratings.

Optionally, the nanostructures may comprise a plasmon resonant material. In particular embodiments, the nanostructures may include a material selected from the group consisting of: Gold (Au), Silver (Ag), Tin (Sn) Rhodium (Rh), Ruthenium (Ru), Palladium (Pd), Osmium (Os), Iridium (Ir), Platinum (Pt), Titanium (Ti) and Aluminum (Al), Chromium (Cr), Copper (Cu), p-type doped silicon, n-type doped silicon, and gallium arsenide.

A structured substrate is provided that includes (a) a plurality of nanoparticles distributed on a solid support; (b) a gel material forming a layer in association with the plurality of nanoparticles; and (c) a library of target nucleic acids in the gel material.

The gel material may cover the nanoparticles.

The solid support may include a surface of a flow cell.

The solid support may include a planar surface having a plurality of wells, the nanoparticles distributed within the plurality of wells.

## Claims

1. An array, comprising:
a solid support comprising a surface, the surface comprising a plurality of reaction cavities, the cavities being separated from each other by interstitial regions;
a plurality of plasmonic nanostructures, wherein the plasmonic nanostructures comprise nanoparticles separated by a controlled spacing, wherein the plasmonic nanostructures form an ensamble amplifier positioned within each of the reaction cavity and configured to amplify at least one of the electromagnetic waves that propagates into the corresponding reaction cavity or amplify electromagnetic energy that is generated within the corresponding reaction cavity;
a gel material forming a layer on the plurality of plasmonic nanostructures; and
a primer nucleic acid attached to the gel material.

2. The array according to claim 1, wherein each of the plasmonic nanostructures comprises a material selected from the group consisting of Gold (Au), Silver (Ag), Tin (Sn), Rhodium (Rh), Ruthenium (Ru), Palladium (Pd), Osmium (Os), Iridium (Ir), Platinum (Pt), Titanium (Ti) and Aluminum (Al), Chromium (Cr), Copper (Cu), p-type doped silicon, n-type doped silicon, and gallium arsenide.

3. The array according to claim 1 or claim 2, wherein the plasmonic nanostructures are situated at the bottom of the cavities.

4. The array according to claim 1 or claim 2, wherein the plasmonic nanostructures are situated along the walls of the cavities.

5. The array according to any one of claims 1 to 4, wherein the interstitial regions are substantially devoid of plasmonic nanostructures.

6. The array according to any one of claims 1 to 5, wherein the nanoparticles comprise dimers or trimers within the cavities.

7. The array according to claim 1, wherein
(a) the nanoparticles have a diameter of greater than 10 nm, 20 nm, 30 nm, 40 nm, 50nm, 60 nm, 70 nm, 80 nm, 90 nm or greater than 100 nm, or
(b) the nanoparticles have a diameter of less than 100 nm, 90 nm, 80 nm, 70 nm, 60 nm, 50 nm, 40 nm, 30 nm, 20 nm or less than 10 nm.

8. The array according to any one of claims 1 to 7, wherein:
the spacing between any two nanoparticles is equal to an excitation wavelength propagated into said plurality of reaction cavities, a multiple of the excitation wavelength, or a fraction of the excitation wavelength; or
the spacing between any two nanoparticles is equal to an emission wavelength generated within said plurality of reaction cavities, a multiple of the excitation emission, or a fraction of the emission wavelength.

9. The array according to any one of claims 1 to 8, wherein the gel material comprises a hydrogel.

10. The array according to any one of claims 1 to 9, wherein the solid support comprises a surface of a flow cell.

11. A method of making an array, comprising:
obtaining a solid support comprising a planar surface, the surface comprising a plurality of reaction cavities, the cavities being separated from each other by interstitial regions;
coating a metal film on the solid support, the metal film having a starting thickness;
subjecting the metal film to a thermal annealing process, thereby forming a plurality of plasmonic nanostructures wherein the plasmonic nanostructures comprise nanoparticles separated by a controlled spacing, wherein the plasmonic nanostructures form an ensamble amplifier positioned within each of the reaction cavity and configured to at least one of amplify electromagnetic waves that propagates into the corresponding reaction cavity or amplify electromagnetic energy that is generated within the corresponding reaction cavity, and wherein a size of the plasmonic nanostructures is a function of the starting film thickness;
coating at least a portion of the solid support with a gel material, thereby depositing the gel material in the plurality of cavities; and
attaching a primer nucleic acid to the gel.

12. The method according to claim 11, further comprising polishing the planar surface to substantially remove the plasmonic nanostructures from the interstitial regions and to maintain the plasmonic nanostructures in the cavities, optionally wherein the nanostructures comprise a material selected from the group consisting of: Gold (Au), Silver (Ag), Tin (Sn) Rhodium (Rh), Ruthenium (Ru), Palladium (Pd), Osmium (Os), Iridium (Ir), Platinum (Pt), Titanium (Ti) and Aluminum (Al), Chromium (Cr), Copper (Cu), p-type doped silicon, n-type doped silicon, and gallium arsenide.

13. A method of detecting nucleic acids, comprising:
(a) providing a solid support comprising a planar surface, the surface comprising a plurality of reaction cavities, the cavities being separated from each other by interstitial regions; a plurality of plasmonic nanostructures wherein the plasmonic nanostructures comprise nanoparticles separated by a controlled spacing; wherein the plasmonic nanostructures form an ensamble amplifier positioned within each of the reaction cavity and configured amplify at least one of the electromagnetic waves that propagates into the corresponding reaction cavity or amplify electromagnetic energy that is generated within the corresponding reaction cavity; a gel material forming a layer covering the plurality of plasmonic nanostructures; a primer nucleic acid attached to the gel material; and a library of target nucleic acids that seed individual cavities via interaction with the primer nucleic acid attached to the gel material;
(b) contacting the solid support with at least one fluorescently labeled probe that binds to the target nucleic acids; and
(c) detecting fluorescent signal on the solid support to distinguish the target nucleic acids that bind to the at least one probe;
optionally wherein the planar surface comprises a surface of a flow cell.

14. The method according to claim 13, wherein
(i) the fluorescently labeled probe comprises a fluorescently labeled nucleotide,
(ii) the fluorescently labeled probe comprises a fluorescently labeled oligonucleotide,
(iii) detecting comprises detection of hybridization of an oligonucleotide probe to the target nucleic acids in each cavity, or
(iv) detecting comprises detection of incorporation of a nucleotide or an oligonucleotide probe to target nucleic acids in each cavity.

15. The method according to claim 13, wherein each of the plasmonic nanostructures comprises a material selected from the group consisting of Gold (Au), Silver (Ag), Tin (Sn), Rhodium (Rh), Ruthenium (Ru), Palladium (Pd), Osmium (Os), Iridium (Ir), Platinum (Pt), Titanium (Ti) and Aluminum (Al), Chromium (Cr), Copper (Cu), p-type doped silicon, n-type doped silicon, and gallium arsenide.

## Patentansprüche

1. Ein Array, das Folgendes beinhaltet:
einen festen Träger, der eine Oberfläche beinhaltet, wobei die Oberfläche eine Vielzahl von Reaktionshohlräumen beinhaltet, wobei die Hohlräume durch interstitielle Bereiche voneinander getrennt sind;
eine Vielzahl plasmonischer Nanostrukturen, wobei die plasmonischen Nanostrukturen Nanopartikel beinhalten, die durch einen kontrollierten Abstand getrennt sind, wobei die plasmonischen Nanostrukturen in jedem der Reaktionshohlräume positioniert einen Ensemble-Verstärker bilden und der konfiguriert ist, um mindestens eine der elektromagnetischen Wellen zu verstärken, die sich in den entsprechenden Reaktionshohlraum ausbreitet, oder elektromagnetische Energie zu verstärken, die innerhalb des entsprechenden Reaktionshohlraums erzeugt wird;
ein Gelmaterial, das auf der Vielzahl plasmonischer Nanostrukturen eine Schicht bildet; und
eine an dem Gelmaterial befestigte Primernukleinsäure.

2. Array gemäß Anspruch 1, wobei jede der plasmonischen Nanostrukturen ein Material beinhaltet, das ausgewählt ist aus der Gruppe, bestehend aus Gold (Au), Silber (Ag), Zinn (Sn), Rhodium (Rh), Ruthenium (Ru), Palladium (Pd), Osmium (Os), Iridium (Ir), Platin (Pt), Titan (Ti) und Aluminium (AI), Chrom (Cr), Kupfer (Cu), p-dotiertem Silizium, n-dotiertem Silizium und Galliumarsenid.

3. Array gemäß Anspruch 1 oder Anspruch 2, wobei sich die plasmonischen Nanostrukturen am Boden der Hohlräume befinden.

4. Array gemäß Anspruch 1 oder Anspruch 2, wobei sich die plasmonischen Nanostrukturen entlang der Wände der Hohlräume befinden.

5. Array gemäß einem der Ansprüche 1 bis 4, wobei die interstitiellen Bereiche im Wesentlichen frei von plasmonischen Nanostrukturen sind.

6. Array gemäß einem der Ansprüche 1 bis 5, wobei die Nanopartikel Dimere oder Trimere innerhalb der Hohlräume beinhalten.

7. Array gemäß Anspruch 1, wobei
(a) die Nanopartikel einen Durchmesser von mehr als 10 nm, 20 nm, 30 nm, 40 nm, 50 nm, 60 nm, 70 nm, 80 nm, 90 nm oder mehr als 100 nm aufweisen oder
(b) die Nanopartikel einen Durchmesser von weniger als 100 nm, 90 nm, 80 nm, 70 nm, 60 nm, 50 nm, 40 nm, 30 nm, 20 nm oder weniger als 10 nm aufweisen.

8. Array gemäß einem der Ansprüche 1 bis 7, wobei:
der Abstand zwischen zwei beliebigen Nanopartikeln gleich einer Anregungswellenlänge, die sich in die Vielzahl von Reaktionshohlräumen ausbreitet, einem Vielfachen der Anregungswellenlänge oder einem Bruchteil der Anregungswellenlänge ist; oder
der Abstand zwischen zwei beliebigen Nanopartikeln gleich einer Emissionswellenlänge, die innerhalb der Vielzahl von Reaktionshohlräumen erzeugt wird, einem Vielfachen der Anregungsemission oder einem Bruchteil der Emissionswellenlänge ist.

9. Array gemäß einem der Ansprüche 1 bis 8, wobei das Gelmaterial ein Hydrogel beinhaltet.

10. Array gemäß einem der Ansprüche 1 bis 9, wobei der feste Träger eine Oberfläche einer Durchflusszelle beinhaltet.

11. Ein Verfahren zum Herstellen eines Arrays, das Folgendes beinhaltet:
Erhalten eines festen Trägers, der eine ebene Oberfläche beinhaltet, wobei die Oberfläche eine Vielzahl von Reaktionshohlräumen beinhaltet, wobei die Hohlräume durch interstitielle Bereiche voneinander getrennt sind;
Überziehen des festen Trägers mit einem Metallfilm, wobei der Metallfilm eine Ausgangsdicke aufweist;
Aussetzen des Metallfilms gegenüber einem thermischen Glühprozess, wodurch eine Vielzahl plasmonischer Nanostrukturen gebildet wird, wobei die plasmonischen Nanostrukturen Nanopartikel beinhalten, die durch einen kontrollierten Abstand getrennt sind, wobei die plasmonischen Nanostrukturen in jedem der Reaktionshohlräume positioniert einen Ensemble-Verstärker bilden und der für mindestens eines von Folgendem konfiguriert ist: Verstärken elektromagnetischer Wellen, die sich in den entsprechenden Reaktionshohlraum ausbreiten, oder Verstärken elektromagnetischer Energie, die innerhalb des entsprechenden Reaktionshohlraums erzeugt wird, und wobei eine Größe der plasmonischen Nanostrukturen eine Funktion der Ausgangsfilmdicke ist;
Überziehen mindestens eines Abschnitts des festen Trägers mit einem Gelmaterial, wodurch das Gelmaterial in der Vielzahl von Hohlräumen abgelagert wird; und Befestigen einer Primernukleinsäure an dem Gel.

12. Verfahren gemäß Anspruch 11, das ferner das Polieren der ebenen Oberfläche, um die plasmonischen Nanostrukturen im Wesentlichen aus den interstitiellen Bereichen zu entfernen und die plasmonischen Nanostrukturen in den Hohlräumen zu halten, beinhaltet, wobei die Nanostrukturen optional ein Material beinhalten, das ausgewählt ist aus der Gruppe, bestehend aus Folgendem: Gold (Au), Silber (Ag), Zinn (Sn) Rhodium (Rh), Ruthenium (Ru), Palladium (Pd), Osmium (Os), Iridium (Ir), Platin (Pt), Titan (Ti) und Aluminium (Al), Chrom (Cr), Kupfer (Cu), p-dotiertem Silizium, n-dotiertem Silizium und Galliumarsenid.

13. Ein Verfahren zum Nachweisen von Nukleinsäuren, das Folgendes beinhaltet:
(a) Bereitstellen eines festen Trägers, der eine ebene Oberfläche beinhaltet, wobei die Oberfläche eine Vielzahl von Reaktionshohlräumen beinhaltet, wobei die Hohlräume durch interstitielle Bereiche voneinander getrennt sind; eine Vielzahl plasmonischer Nanostrukturen, wobei die plasmonischen Nanostrukturen Nanopartikel beinhalten, die durch einen kontrollierten Abstand getrennt sind; wobei die plasmonischen Nanostrukturen in jedem der Reaktionshohlräume positioniert einen Ensemble-Verstärker bilden, und der konfiguriert ist, um mindestens eine der elektromagnetischen Wellen zu verstärken, die sich in den entsprechenden Reaktionshohlraum ausbreitet, oder elektromagnetische Energie zu verstärken, die innerhalb des entsprechenden Reaktionshohlraums erzeugt wird; ein Gelmaterial, das eine Schicht bildet, die die Vielzahl plasmonischer Nanostrukturen bedeckt; eine an dem Gelmaterial befestigte Primernukleinsäure und eine Bibliothek von Zielnukleinsäuren, die einzelne Hohlräume durch Wechselwirkung mit der an dem Gelmaterial befestigten Primernukleinsäure animpfen;
(b) In-Kontakt-Bringen des festen Trägers mit mindestens einer fluoreszenzmarkierten Sonde, die an die Zielnukleinsäuren bindet; und
(c) Nachweisen eines Fluoreszenzsignals auf dem festen Träger, um die Zielnukleinsäuren zu unterscheiden, die an die mindestens eine Sonde binden; wobei die ebene Oberfläche optional eine Oberfläche einer Durchflusszelle beinhaltet.

14. Verfahren gemäß Anspruch 13, wobei
(i) die fluoreszenzmarkierte Sonde ein fluoreszenzmarkiertes Nukleotid beinhaltet,
(ii) die fluoreszenzmarkierte Sonde ein fluoreszenzmarkiertes Oligonukleotid beinhaltet,
(iii) das Nachweisen den Nachweis der Hybridisierung einer Oligonukleotidsonde mit den Zielnukleinsäuren in jedem Hohlraum beinhaltet oder
(iv) das Nachweisen den Nachweis des Inkorporierens eines Nukleotids oder einer Oligonukleotidsonde in Zielnukleinsäuren in jedem Hohlraum beinhaltet.

15. Verfahren gemäß Anspruch 13, wobei jede der plasmonischen Nanostrukturen ein Material beinhaltet, das ausgewählt ist aus der Gruppe, bestehend aus Gold (Au), Silber (Ag), Zinn (Sn), Rhodium (Rh), Ruthenium (Ru), Palladium (Pd), Osmium (Os), Iridium (Ir), Platin (Pt), Titan (Ti) und Aluminium (Al), Chrom (Cr), Kupfer (Cu), p-dotiertem Silizium, n-dotiertem Silizium und Galliumarsenid.

## Revendications

1. Un réseau, comprenant :
un support solide comprenant une surface, la surface comprenant une pluralité de cavités de réaction, les cavités étant séparées les unes des autres par des régions interstitielles ;
une pluralité de nanostructures plasmoniques, les nanostructures plasmoniques comprenant des nanoparticules séparées par un espacement contrôlé, les nanostructures plasmoniques formant un amplificateur d'ensemble positionné au sein de chaque cavité de réaction et configuré pour amplifier au moins une des ondes électromagnétiques qui se propage dans la cavité de réaction correspondante ou amplifier l'énergie électromagnétique qui est générée au sein de la cavité de réaction correspondante ;
un matériau gel formant une couche sur la pluralité de nanostructures plasmoniques ; et
un acide nucléique amorce attaché au matériau gel.

2. Le réseau selon la revendication 1, dans lequel chacune des nanostructures plasmoniques comprend un matériau sélectionné dans le groupe constitué d'or (Au), d'argent (Ag), d'étain (Sn), de rhodium (Rh), de ruthénium (Ru), de palladium (Pd), d'osmium (Os), d'iridium (Ir), de platine (Pt), de titane (Ti) et d'aluminium (Al), de chrome (Cr), de cuivre (Cu), de silicium dopé de type p, de silicium dopé de type n, et d'arséniure de gallium.

3. Le réseau selon la revendication 1 ou la revendication 2, dans lequel les nanostructures plasmoniques sont situées au fond des cavités.

4. Le réseau selon la revendication 1 ou la revendication 2, dans lequel les nanostructures plasmoniques sont situées le long des parois des cavités.

5. Le réseau selon n'importe laquelle des revendications 1 à 4, dans lequel les régions interstitielles sont substantiellement dépourvues de nanostructures plasmoniques.

6. Le réseau selon n'importe laquelle des revendications 1 à 5, dans lequel les nanoparticules comprennent des dimères ou trimères au sein des cavités.

7. Le réseau selon la revendication 1, dans lequel
(a) les nanoparticules ont un diamètre supérieur à 10 nm, 20 nm, 30 nm, 40 nm, 50 nm, 60 nm, 70 nm, 80 nm, 90 nm ou supérieur à 100 nm, ou
(b) les nanoparticules ont un diamètre inférieur à 100 nm, 90 nm, 80 nm, 70 nm, 60 nm, 50 nm, 40 nm, 30 nm, 20 nm ou inférieur à 10 nm.

8. Le réseau selon n'importe laquelle des revendications 1 à 7, dans lequel :
l'espacement entre deux nanoparticules quelconques est égal à une longueur d'onde d'excitation propagée dans ladite pluralité de cavités de réaction, un multiple de la longueur d'onde d'excitation, ou une fraction de la longueur d'onde d'excitation ; ou
l'espacement entre deux nanoparticules quelconques est égal à une longueur d'onde d'émission générée au sein de ladite pluralité de cavités de réaction, un multiple de l'émission d'excitation, ou une fraction de la longueur d'onde d'émission.

9. Le réseau selon n'importe laquelle des revendications 1 à 8, dans lequel le matériau gel comprend un hydrogel.

10. Le réseau selon n'importe laquelle des revendications 1 à 9, dans lequel le support solide comprend une surface d'une cuve à circulation (*flow cell*).

11. Un procédé de fabrication d'un réseau, comprenant :
le fait d'obtenir un support solide comprenant une surface plane, la surface comprenant une pluralité de cavités de réaction, les cavités étant séparées les unes des autres par des régions interstitielles ;
le fait de revêtir un film de métal sur le support solide, le film de métal ayant une épaisseur de départ ;
le fait de soumettre le film de métal à un processus d'annelage thermique, formant de ce fait une pluralité de nanostructures plasmoniques, les nanostructures plasmoniques comprenant des nanoparticules séparées par un espacement contrôlé, les nanostructures plasmoniques formant un amplificateur d'ensemble positionné au sein de chaque cavité de réaction et configuré au moins soit pour amplifier des ondes électromagnétiques qui se propagent dans la cavité de réaction correspondante, soit pour amplifier l'énergie électromagnétique qui est générée au sein de la cavité de réaction correspondante, et une taille des nanostructures plasmoniques étant fonction de l'épaisseur de film de départ ;
le fait de revêtir au moins une portion du support solide avec un matériau gel, déposant de ce fait le matériau gel dans la pluralité de cavités ; et le fait d'attacher un acide nucléique amorce au gel.

12. Le procédé selon la revendication 11, comprenant en outre le fait de polir la surface plane afin d'éliminer substantiellement les nanostructures plasmoniques des régions interstitielles et afin de maintenir les nanostructures plasmoniques dans les cavités, facultativement dans lequel les nanostructures comprennent un matériau sélectionné dans le groupe constitué : d'or (Au), d'argent (Ag), d'étain (Sn), de rhodium (Rh), de ruthénium (Ru), de palladium (Pd), d'osmium (Os), d'iridium (Ir), de platine (Pt), de titane (Ti) et d'aluminium (Al), de chrome (Cr), de cuivre (Cu), de silicium dopé de type p, de silicium dopé de type n, et d'arséniure de gallium.

13. Un procédé de détection d'acides nucléiques, comprenant :
(a) le fait de fournir un support solide comprenant une surface plane, la surface comprenant une pluralité de cavités de réaction, les cavités étant séparées les unes des autres par des régions interstitielles ; une pluralité de nanostructures plasmoniques, les nanostructures plasmoniques comprenant des nanoparticules séparées par un espacement contrôlé ; les nanostructures plasmoniques formant un amplificateur d'ensemble positionné au sein de chaque cavité de réaction et configuré pour amplifier au moins une des ondes électromagnétiques qui se propage dans la cavité de réaction correspondante ou amplifier l'énergie électromagnétique qui est générée au sein de la cavité de réaction correspondante ; un matériau gel formant une couche recouvrant la pluralité de nanostructures plasmoniques ; un acide nucléique d'amorce attaché au matériau gel ; et une bibliothèque d'acides nucléiques cibles qui ensemence des cavités individuelles par le biais d'une interaction avec l'acide nucléique d'amorce attaché au matériau gel ;
(b) le fait de mettre en contact le support solide avec au moins une sonde marquée par fluorescence qui se lie aux acides nucléiques cibles ; et
(c) le fait de détecter un signal fluorescent sur le support solide afin de distinguer les acides nucléiques cibles qui se lient à l'au moins une sonde ; facultativement dans lequel la surface plane comprend une surface d'une cuve à circulation.

14. Le procédé selon la revendication 13, dans lequel :
(i) la sonde marquée par fluorescence comprend un nucléotide marqué par fluorescence,
(ii) la sonde marquée par fluorescence comprend un oligonucléotide marqué par fluorescence,
(iii) le fait de détecter comprend la détection de l'hybridation d'une sonde d'oligonucléotide aux acides nucléiques cibles dans chaque cavité, ou
(iv) le fait de détecter comprend la détection de l'incorporation d'un nucléotide ou d'une sonde d'oligonucléotide aux acides nucléiques cibles dans chaque cavité.

15. Le procédé selon la revendication 13, dans lequel chacune des nanostructures plasmoniques comprend un matériau sélectionné dans le groupe constitué d'or (Au), d'argent (Ag), d'étain (Sn), de rhodium (Rh), de ruthénium (Ru), de palladium (Pd), d'osmium (Os), d'iridium (Ir), de platine (Pt), de titane (Ti) et d'aluminium (Al), de chrome (Cr), de cuivre (Cu), de silicium dopé de type p, de silicium dopé de type n, et d'arséniure de gallium.
